# EUROPEAN PATENT SPECIFICATION

(11) **EP 4 326 312 B1**
(45) Date of publication and mention of the grant of the patent: **16.09.2026**
(21) Application number: 22716982.8
(22) Date of filing: 14.04.2022
(51) Int. Cl.: A61K 38/30, A61K 47/66, A61P 13/10, C07K 14/65

(54) **TREATMENT OF LOWER URINARY TRACT SYMPTOMS**
BEHANDLUNG VON SYMPTOMEN DER UNTEREN HARNWEGE
TRAITEMENT DE SYMPTÔMES DE LA VOIE URINAIRE INFÉRIEURE

(30) Priority: 19.04.2021 EP 21169118
(43) Date of publication of application: 28.02.2024
(73) Proprietor: Versameb AG, 4057 Basel (CH)
(72) Inventor: ZUIDEVELD, Klaas Pieter, 4125 Riehen (CH); SELVARAJ, Justin Antony, 79576 Weil am Rhein (DE); HILLMANN-WÜLLNER, Petra, 8049 Zürich (CH); METZGER, Friedrich, 79115 Freiburg (DE)
(74) Representative: Jeck, Jonathan
(86) International application number: PCT/EP2022/060005
(87) International publication number: WO 2022/223428

(56) References cited:
- WO-A1-2009/046739
- WO-A1-98/33529
- WO-A1-99/10013
- WO-A2-2020/127532
- WO-A2-2021/130537
- YAN HAO ET AL: "Controlled release of insulin-like growth factor 1 enhances urethral sphincter function and histological structure in the treatment of female stress urinary incontinence in a rat model", vol. 121, no. 2, 1 February 2018 (2018-02-01), pages 301 - 312, XP055843327, ISSN: 1464-4096, Retrieved from the Internet <URL:https://www.ncbi.nlm.nih.gov/pmc/articles/PMC8121163/pdf/nihms-1701624.pdf> DOI: 10.1111/bju.13985
- SUMINO YASUHIRO ET AL: "Therapeutic Effects of IGF-1 on Stress Urinary Incontinence in Rats with Simulated Childbirth Trauma", JOURNAL OF UROLOGY, LIPPINCOTT WILLIAMS & WILKINS, BALTIMORE, MD, US, vol. 191, no. 2, 12 September 2013 (2013-09-12), pages 529 - 538, XP028813278, ISSN: 0022-5347, DOI: 10.1016/J.JURO.2013.08.109
- WALLIS ET AL: "New insulin-like growth factor (IGF)-precursor sequences from mammalian genomes: the molecular evolution of IGFs and associated peptides in primates", GROWTH HORMONE AND IGF RESEARCH, CHURCHILL LIVINGSTONE, LONDON, GB, vol. 19, no. 1, 1 February 2009 (2009-02-01), pages 12 - 23, XP025892487, ISSN: 1096-6374, [retrieved on 20080620], DOI: 10.1016/J.GHIR.2008.05.001

## Description

### The field of the invention

The present invention relates to a mRNA and a therapeutic composition thereof for use in a method of treating lower urinary tract symptoms (LUTS).

### Background of the invention

Lower urinary tract symptoms (LUTS, MeSH Tree Number: C23.888.942.343, MeSH Unique ID: D059411) include frequency, nocturia, urgency, incomplete voiding and urinary incontinence. They are often associated with diseases such as stress urinary incontinence (SUI), urge urinary incontinence, mixed urinary incontinence, under active bladder, overflow incontinence and pelvic organ prolapse. Treatment options for LUTS are limited to conservative approaches such as life style changes (eg loss of weight, quiting smoking), the use of incontinence pads or liners and physical therapy (pelvic floor exerices, the use of inserts or pelvic floor strengthening and nerve stimulation products), with poor compliance and limited efficacy or invasive procedures aush as surgery (midurethral mesh and non-synthetic sling procedures or bladder neck suspension sling procedures) and the inection of bulking agents to restore continence with mixed outcome and non-reversible consequences in case of failed procedures (for an extensive description see Takahashi et al., Clinical Guideline for Female Lower Urinary Tract Symptoms. Low Urin Tract Symptoms, 2016 Jan;8(1):5-29). Hence, with no effective and safe pharmaceutical therapies available today for treating LUTS, there is a high unmet medical need for therapies which restore the function of the urethra, urinary sphincter, and bladder.

### Summary of the invention

The present invention provides a mRNA comprising a nucleic acid sequence encoding insulin-like growth factor 1 (IGF1) and a therapeutic composition thereof for use in a method of treating lower urinary tract symptoms. The present inventors have surprisingly found that in animals treated with mRNA encoding IGF1 the therapeutic effect in relation to lower urinary tract symptoms of the mRNA administered lasts for nine days or more after administration of the mRNA and protected the urinary tract tissue from degeneration.

### Brief description of the figures

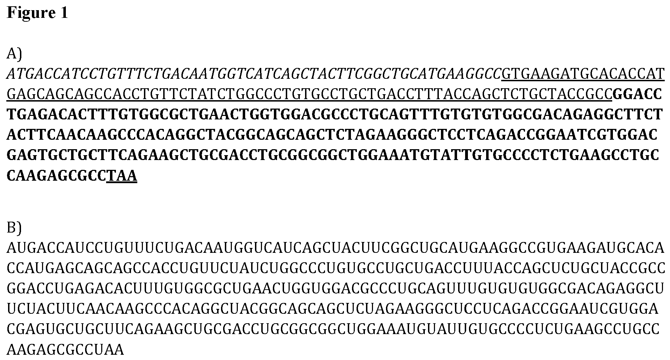

**Figure 1** shows DNA and RNA sequence of Cpd.1A and Cpd.1B, which are identical. **(A)** shows the DNA sequence (SEQ ID NO: 7) of human codon-optimized IGF1 containing the BDNF pre-domain (signalling peptide) and IGF1 pro- and coding domains. The sequence for the pre-domain (signalling peptide) is indicated in *italic,* the sequence for the pro-domain is underlined, the IGF1 coding domain is indicated in **bold** and the stop codon in **bold. (B)** illustrates the RNA sequence of BDNF pre- and IGF1 pro- and coding domains (SEQ ID NO: 8), wherein uridine is N1-Methylpseudouridine. Pre- and pro-domains are cleaved of upon secretion.

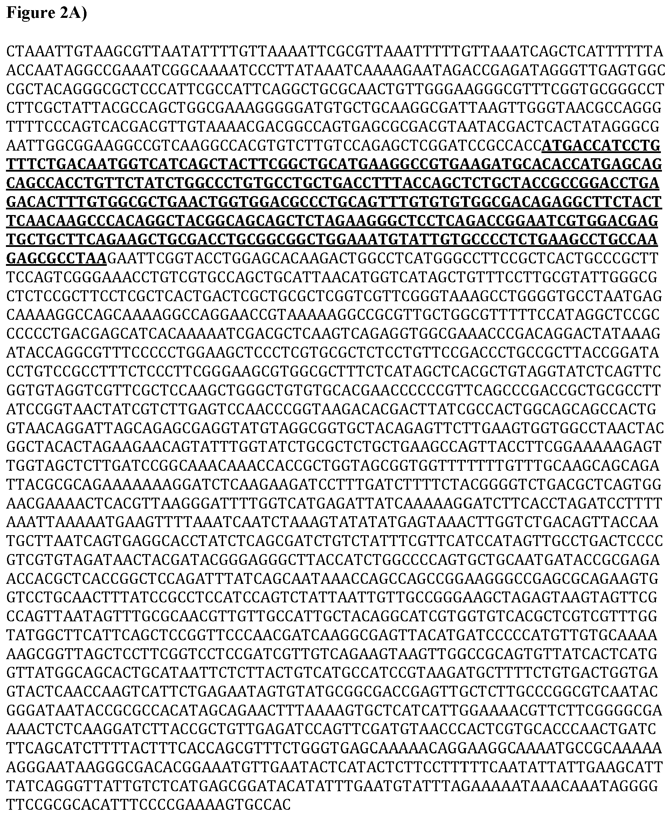

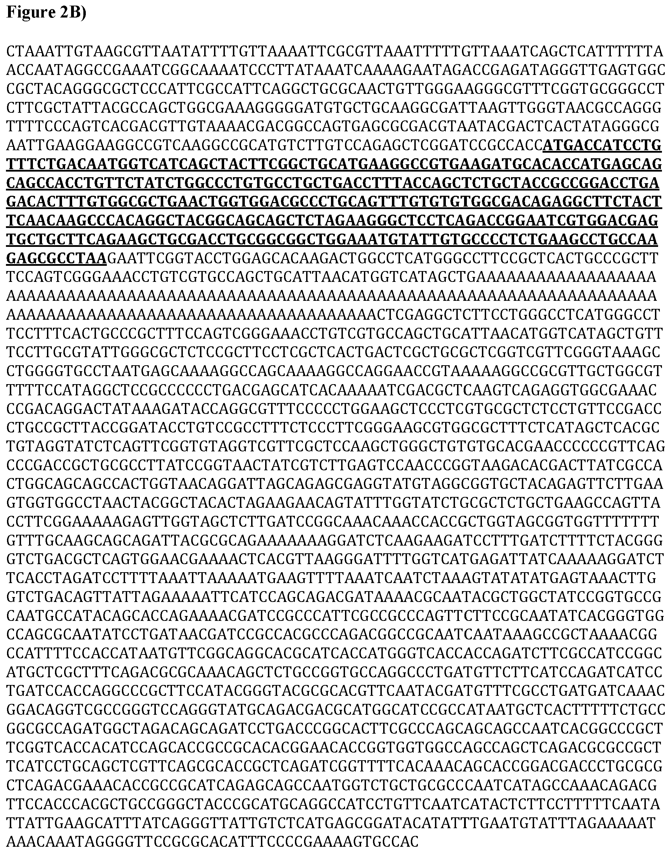

Figure 2 **A)** shows the DNA sequence of vector pMA-T with Cpd.1A insert marked in **bold** (SEQ ID NO: 9). Figure 2B) shows the DNA sequence of vector pMK with Cpd.1B insert marked in **bold** (SEQ ID NO: 10).

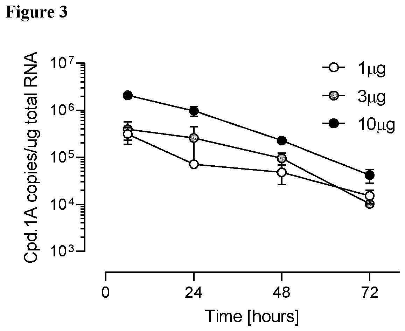

**Figure 3** shows Pharmacokinetics of Cpd.1A in TA muscle after i.m. injury. Cpd.1A was injected into TA muscle at 1, 3 and 10 µg single doses, and muscle samples analyzed for Cpd.1A mRNA at different time points. Data show near linear tissue distribution in TA muscle with a relevant exposure of Cpd.1A within muscle over up to 72 hours after injection. Data represent mean ± SEM of 8 repetitions per data point.

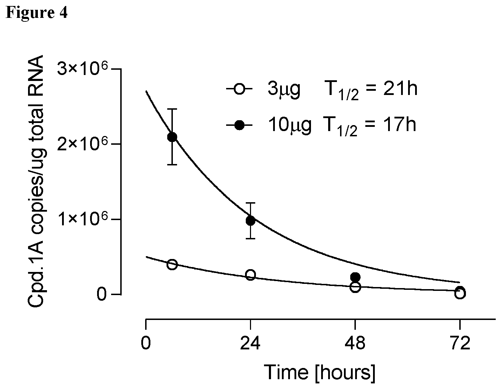

**Figure 4** shows monoexponential curves derived for half-life calculation of Cpd.1A in TA muscle after i.m. injection. Cpd.1A was injected into TA muscle at 3 and 10 µg single doses, and the time course of Cpd.1A decline was analyzed . The half-life of 17-21 hours (for both doses) follows a mono-exponential time course and confirms a relevant exposure of Cpd.1A over up to 72 hours after injection. Data represent mean ± SEM of 8 repetitions per data point.

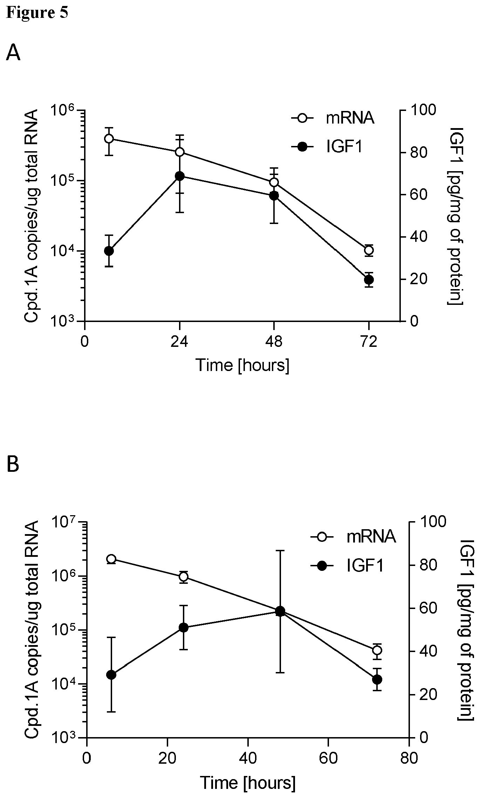

**Figure 5** shows Cpd.1A mRNA (A: 3 µg dose, B: 10 µg dose) vs. IGF1 protein expression in TA muscle after i.m. injection. Cpd.1A mRNA and IGF1 protein levels were assessed after different time points. Data confirm that after a delay of several hours, tissue starts to produce IGF1 protein at functionally relevant concentrations over up to 72 hours after injection. Data represent mean ± SEM of 8 repetitions per data point.

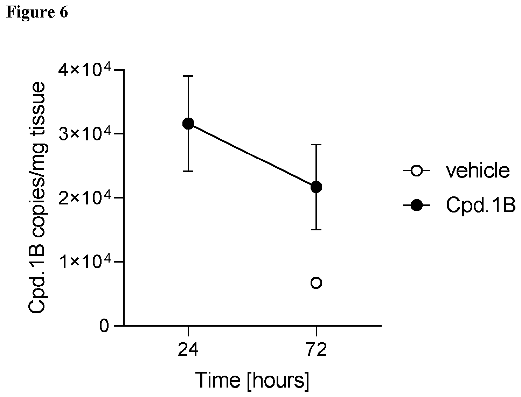

**Figure 6** shows Pharmacokinetics of Cpd.1B in urethra after i.m. injection. Cpd.1B was injected into urethral tissue as a 30 µg single dose, and exposure analyzed at 24 and 72 hours after injection. Data indicate significant exposure of Cpd.1B mRNA over up to 72 hours. Data represent mean ± SEM of 3 repetitions per data point.

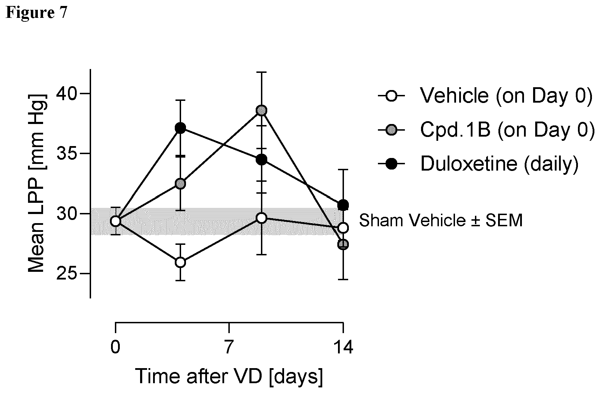

**Figure** 7 shows Leak point pressure of the urinary bladder after vaginal distension. Rats were anesthetized on Day 0 and vaginal distension (VD) induced by a balloon catheter. 4 hours after VD, Cpd.1B or vehicle into the urethral muscle as single dose. As positive control, duloxetine was given orally daily during the entire experiment. On Day 4, 9 and 14 after VD, rats were investigated for leak point pressure (LPP) defining urinary incontinence. Vehicle treated rats showed a reduction in LPP on Day 4 and a full recovery to normal levels without VD by Day 9 and 14 (Sham vehicle). Cpd.1B treated rats showed an increase of LPP over time that returned to normal levels by Day 14. Duloxetine showed an initial increase in LPP that declined thereafter to normal levels on Day 14. The data suggest a regenerative mechanism of IGF1 mediated muscle functional creating sustained improvement over time, and the return to normal functional levels on Day 14 indicates no IGF1 mediated tissue hypertrophy. In contrast Duloxetine showed a different time course of efficacy with an initial response that declined over time, differentiating IGF1 mRNA mechanistically from Duloxetine. Data represent mean ± SEM of 10 animals per time point and group.

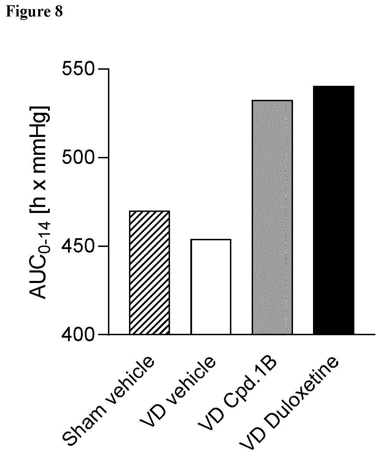

**Figure 8** shows Area under curve (AUC) of urinary bladder leak point pressure after vaginal distension over the entire time course of LPP from Day 0-14. Data support beneficial efficacy of Cpd.1B and Duloxetine. Data represent average AUC of time course data analyzed in Figure 7.

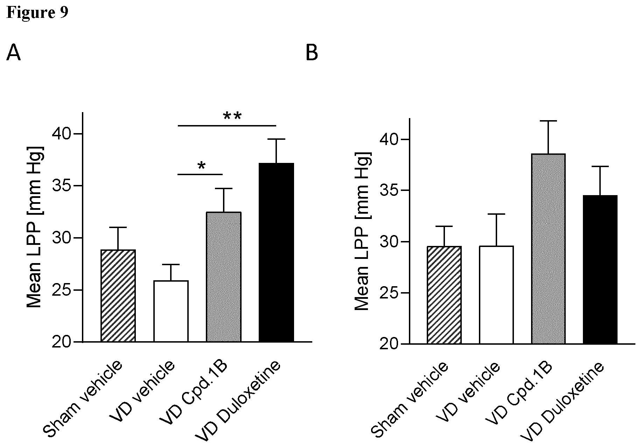

**Figure 9** shows Urinary bladder leak point pressure on days 4 and 9 after vaginal distension (VD). VD decreases LPP on Day 4 (A) whereas both Cpd.1B single injection on Day 0 or daily treatment with Duloxetine increased LPP. On Day 9 (B), LPP had recovered in the VD vehicle group to normal levels, whereas Cpd.1B and Duloxetine still showed increased LPP. Data represent mean ± SEM of 10 animals per group and time point. *, p<0.05, **, p<0.01 vs. VD vehicle as assessed by Student's t-test.

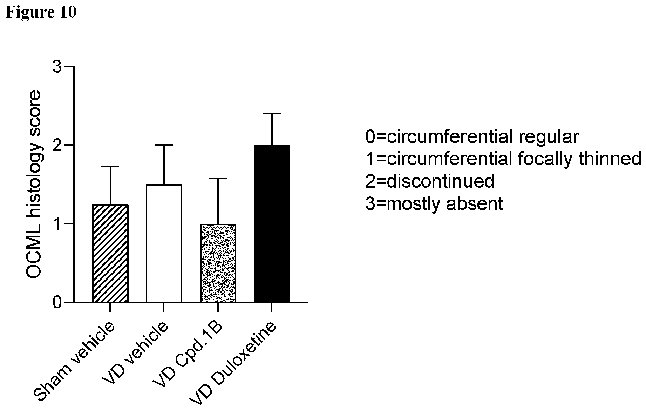

**Figure 10** shows histology results of the outer circular muscle layer (OCML) from cross-sections of mid-urethra after Masson trichrome staining. The OMCL consists of a thin layer of skeletal muscle cells surrounding the urethra. Under normal conditions, the OMCL skeletal muscle cells are well organized without connective tissue. VD showed a trend for increasing the OCML score towards a higher distortion of the OCML layer, an effect which is reverted by Cpd.1B. In contrast duloxetine treatment tends to worsen the OCML disorganization. The data suggest a beneficial therapeutic effect of Cpd.1B but not duloxetine on skeletal muscle preservation and regeneration in the OCML. Data represent mean ± SEM of 4 repetitions per data point.

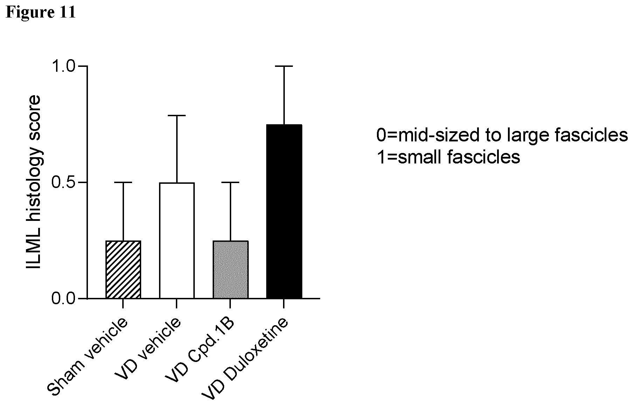

**Figure 11** shows histology results of the inner longitudinal muscle layer (ILML) from cross-sections of mid-urethra after Masson trichrome staining. The ILML consists of layers of smooth muscle cells at the inner edge of the urethra. Under normal conditions, the OMCL skeletal muscle cells are well organized in fascicles without connective tissue. VD increases the ILML score towards increased tissue distortion and fascicle disruption, an effect which is reverted by Cpd.1B. In contrast duloxetine treatment worsens the ILML score indicating further disorganization of smooth muscle fascicles. The data suggest a beneficial therapeutic effect of Cpd.1B but not duloxetine on smooth muscle preservation and regeneration. Data represent mean ± SEM of 4 repetitions per data point.

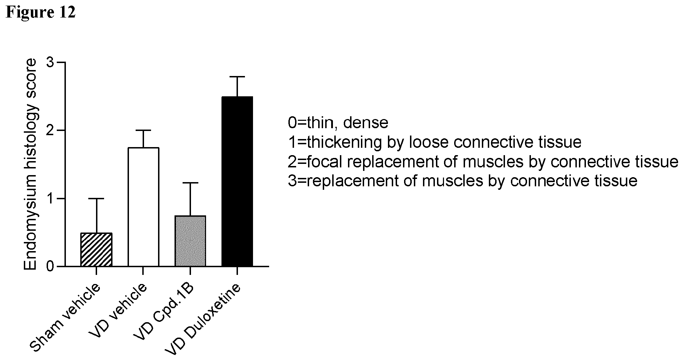

**Figure 12** shows histology results of the endomysial tissue from cross-sections of mid-urethra after Masson trichrome staining. The staining allows to illustrate the amount of connective tissue including collagen fibers between muscle fibers (mainly in the OCML and ILML). This endomysial tissue (or endomysium) is dense under normal conditions, after VD diffusely thickened to discontinue the skeletal (OCML) and smooth muscle (ILML) layers OCML, and focally replaces muscle by connective tissue in most extreme disease stage. VD increases of the Endomysium score towards higher amounts of connective tissue and muscle replacement, an effect which is reverted by Cpd.1B. In contrast duloxetine treatment worsens the endomysium score and thus does not prevent disorganization of the muscle layers and infiltration of connective tissue. Data represent mean ± SEM of 4 repetitions per data point.

### Detailed description of the invention

The term "RNA" as used herein includes RNA which codes for an amino acid sequence. Usually the RNA as used herein is a coding RNA, i.e. an RNA which codes for an amino acid sequence. Such RNA molecules are also referred to as mRNA (messenger RNA) and are single-stranded RNA molecules. Thus the term "RNA" as used herein preferably refers to mRNA. The RNA may be made by synthetic chemical and enzymatic methodology known to one of ordinary skill in the art, or by the use of recombinant technology, or may be isolated from natural sources, or by a combination thereof. The RNA may optionally comprise unnatural and naturally occurring nucleoside modifications such as e.g. N¹-Methylpseudouridine also referred herein as methylpseudouridine.

The term "mRNA" (i.e. messenger RNA) as used herein refers to polymers which are built up of nucleoside phosphate building blocks mainly with adenosine, cytidine, uridine and guanosine as nucleosides, and which contain a coding region encoding a protein. In the context of the present invention, mRNA should be understood to mean any polyribonucleotide molecule which, if it comes into the cell, is suitable for the expression of a protein or fragment thereof or is translatable to a protein or fragment thereof. The mRNA of the present invention comprising a nucleic acid sequence encoding a protein and a signal peptide should be understood to mean a polyribonucleotide molecule which, if it comes into the cell, is suitable to induce the expression and secretion of said protein or fragment thereof. The mRNA of the present invention is an artificial nucleic acid molecule, i.e. an artificial mRNA. An artificial nucleic acid molecule e.g. an artificial mRNA may typically be understood to be a nucleic acid molecule, that does not occur naturally, like a recombinant mRNA. A recombinant mRNA is the preferred mRNA of the present invention. The mRNA contains a ribonucleotide sequence which encodes a protein or fragment thereof whose function in the cell or in the vicinity of the cell is usually needed or beneficial, in particular in the context of the healing of lower urinary tract symptoms. The mRNA may contain the sequence for the complete protein or a functional variant thereof. Thus the nucleic acid sequence of the mRNA for the complete protein usually comprises a nucleic acid sequence encoding the signal peptide and a nucleic acid sequence encoding the protein. The mRNA of the present invention comprises a nucleic acid sequence encoding a protein and a signal peptide. The nucleic acid sequence encoding a protein may optionally comprise the pro-domain of a protein, which is usually located at the N-terminus of the protein. The protein and the signal peptide are usually encoded by the nucleic acid sequence of the mRNA of the present invention in the following order from 5' to 3': i) the signal peptide and ii) the protein i.e the last nucleoside of the coding region of the signal peptide is followed by the first nucleoside of the coding region of the protein or in case of a protein comprising a pro-domain by the first nucleoside of the coding region of the pro-protein form of the protein. The ribonucleotide sequence can encode a protein which acts as a factor, inducer, regulator, stimulator or enzyme, or a functional fragment thereof, where this protein is usually one whose function is necessary in order to remedy a disorder, in particular lower urinary tract symptoms. Here, functional variant is understood to mean a fragment which in the cell can undertake the function of the protein whose function in the cell is needed. In addition, the mRNA may also have further functional regions and/or 3' or 5' noncoding regions. The 3' and/or 5' noncoding regions can be the regions naturally flanking the protein-encoding sequence or artificial sequences which contribute to the stabilization of the RNA like e.g. a cap at the 5' end and/or a polyA tail at the 3' end. Those skilled in the art can determine the sequences suitable for this in each case by routine experiments. The mRNA or the DNA used to transcribe the mRNA may be codon optimized. Preferably, the DNA used in the present invention to transcribe the mRNA of the present invention and the mRNA of the present invention are codon optimized. In general, codon optimization refers to a process of modifying a nucleic acid sequence for expression in a host cell of interest by replacing at least one codon (e.g. more than 1 , 2, 3, 4, 5, 10, 15, 20, 25, 50, or more codons) of a native sequence with codons that are more frequently or most frequently used in the genes of that host cell while maintaining the native amino acid sequence. Codon usage tables are readily available, for example, at the "Codon Usage Database", and these tables can be adapted in a number of ways. Computer algorithms for codon optimizing a particular sequence for expression in a particular host cell are also available, such as Gene Forge^{®} (Aptagen, PA) and GeneOptimizer^{®} (ThermoFischer, MA) which is preferred.

The term "naked RNA" as used herein refers to an RNA which is not complexed to any kind of other compound, in particular proteins, peptides, polymers, like cationic polymers, lipids, liposomes, viral vectors or the like. Thus, "naked RNA" means that the RNA is present e.g. in a liquid composition in a free and uncomplexed form being molecularly dispersed in solution. For example, it is not envisaged that the "naked RNA" is complexed with a lipid and/or polymer carrier system (e.g., lipid nano particles and micelle)/transfection reagent like, for example, DreamFect^{™} Gold or (branched) PEI. Hence, a composition comprising the mRNA, like the therapeutic composition of the invention, does, for example, not contain a lipid and/or polymer carrier system transfection reagent like, for example, DreamFect^{™} Gold or (branched) PEI.

The terms "nucleic acid sequence", "nucleotide sequence" and "nucleotide acid sequence" are used herein interchangeably and have the identical meaning herein, and refer to preferably DNA or RNA. The terms "nucleic acid sequence", "nucleotide sequence" and "nucleotide acid sequence" are preferably used synonymous with the term "polynucleotide sequence". Preferably, a nucleic acid sequence is a polymer comprising or consisting of nucleotide monomers, which are covalently linked to each other by phosphodiester-bonds of a sugar/phosphate-backbone. The term "nucleic acid sequence" also encompasses modified nucleic acid sequences, such as base-modified, sugar-modified or backbone-modified etc. DNA or RNA.

The term "open reading frame" as used herein refers to a sequence of several nucleotide triplets, which may be translated into a peptide or protein. An open reading frame (ORF) preferably contains a start codon, i.e. a combination of three subsequent nucleotides coding usually for the amino acid methionine (ATG), at its 5'-end and a subsequent region, which usually exhibits a length which is a multiple of 3 nucleotides. An ORF is preferably terminated by a stop-codon (e.g., TAA, TAG, TGA). Typically, this is the only stop-codon of the open reading frame. Thus, an open reading frame in the context of the present invention is preferably a nucleic acid sequence, consisting of a number of nucleotides that may be divided by three, which starts with a start codon (e.g. ATG) and which preferably terminates with a stop codon (e.g., TAA, TGA, or TAG). The open reading frame may be isolated or it may be incorporated in a longer nucleic acid sequence, for example in a vector or an mRNA. An open reading frame may also be termed "(protein) coding region" or, preferably, "coding sequence".

The term "signal peptide" also referred herein to as signalling peptide, pre-domain, signal sequence, targeting signal, localization signal, localization sequence, transit peptide, leader sequence or leader peptide is a short peptide (usually 16-40 amino acids long) present at the N-terminus of newly synthesized proteins that are destined towards the secretory pathway. The signal peptide of the present invention is preferably 10-50, more preferably 11-45, even more preferably 12-45, most preferably 13-45, in particular 14-45, more particular 15-45, even more particular 16-40 amino acids long. A signal peptide according to the invention is situated at the N-terminal end of the protein of interest or at at the N-terminal end of the pro-protein form of the protein of interest. A signal peptide according to the invention is usually of eukaryotic origin e.g. the signal peptide of a eukaryotic protein, preferably of mammalian origin e.g. the signal peptide of a mammalian protein, more preferably of human origin e.g. the signal peptide of a mammalian protein. In some embodiments the heterologous signal peptide and/or the homologous signal peptide to be modified is the naturally occurring signal peptide of a eukaryotic protein, preferably the naturally occurring signal peptide of a mammalian protein, more preferably the naturally occurring signal peptide of a human protein.

The term "protein" as used herein refers to molecules typically comprising one or more peptides or polypeptides. A peptide or polypeptide is typically a chain of amino acid residues, linked by peptide bonds. A peptide usually comprises between 2 and 50 amino acid residues. A polypeptide usually comprises more than 50 amino acid residues. A protein is typically folded into 3-dimensional form, which may be required for the protein to exert its biological function. The term "protein" as used herein includes a fragment of a protein and fusion proteins. Preferably the protein is of mammalian, more preferably human origin i.e. is a human protein. Preferably the protein is a protein which is normally secreted from a cell, i.e. a protein which is secreted from a cell in nature. Proteins as refered herein are preferably growth factors. Growth factors are secreted proteins capable of stimulating cellular growth, proliferation, healing, and cellular differentiation either acting locally or systemically as modulators of target cell signalling via receptors on their surfaces, often involved in trophic reactions and survival or cell homeostasis signalling. Growth factors as referred herein can be found in the UniProt database.

The term "fragment" or "fragment of a sequence" which have the identical meaning herein is a shorter portion of a full-length sequence of e.g. a nucleic acid molecule like DNA or RNA or a protein. Accordingly, a fragment, typically, comprises or consists of a sequence that is identical to the corresponding stretch within the full-length sequence. A preferred fragment of a sequence in the context of the present invention, comprises or consists of a continuous stretch of entities, such as nucleotides or amino acids corresponding to a continuous stretch of entities in the molecule the fragment is derived from, which represents at least 5%, usually at least 20%, preferably at least 30%, more preferably at least 40%, more preferably at least 50%, even more preferably at least 60%, even more preferably at least 70%, and most preferably at least 80% of the total (i.e. full-length) molecule, from which the fragment is derived.

The term "signal peptide heterologous to said protein" as used herein refers to a naturally occurring signal peptide which is different to the naturally occurring signal peptide of the protein, i.e. the signal peptide is not derived from the same gene of the protein. Usually a signal peptide heterologous to a given protein is a signal peptide from another protein, which is not related to the given protein i.e. which has an amino acid sequence which differs from the signal peptide of the given protein, e.g. which has an amino acid sequence which differs from the signal peptide of the given protein by more than 50%, preferably by more than 60%, more preferably by more than 70%, even more preferably by more than 80%, most preferably by more than 90%, in particular by more than 95%. Preferably a signal peptide heterologous to a given protein has a sequence identity with the amino acid sequence of the naturally occurring (homologous) signal peptide of the given protein of less than 95%, preferably less than 90%, more preferably less than 80%, even more preferably less than 70%, most preferably less than 60%, in particular less than 50%. Although heterologous sequences may be derivable from the same organism, they naturally (in nature) do not occur in the same nucleic acid molecule, such as in the same mRNA. The signal peptide heterologous to a protein and the protein to which the signal peptide is heterologous can be of the same or different origin and are usually of the same origin, preferably of eukaryotic origin, more preferably of eukaryotic origin of the same eukaryotic organism, even more preferably of mammalian origin, in particular of mammalian origin of the same mammalian organism, more particlular of human origin. In Example 1 a mRNA comprising a nucleic acid sequence encoding the human BDNF signal peptide and the human IGF1, i.e. a signal peptide heterologous to a protein wherein the signal peptide and the protein are of the same origin, namely of human origin is disclosed.

The term "signal peptide homologous to said protein" as used herein refers to the naturally occurring signal peptide of a protein. A signal peptide homologous to a protein is the signal peptide encoded by the gene of the protein as it occurs in nature. A signal peptide homologous to a protein is usually of eukaryotic origin e.g. the naturally occurring signal peptide of a eukaryotic protein, preferably of mammalian origin e.g. the naturally occurring signal peptide of a mammalian protein, more preferably of human origin e.g. the naturally occurring signal peptide of a human protein.

The term "naturally occurring amino acid sequence which does not have the function of a signal peptide in nature" as used herein refers to an amino acid sequence which occurs in nature and which is not identical to the amino acid sequence of any signal peptide occurring in nature. The naturally occurring amino acid sequence which does not have the function of a signal peptide in nature as referred to in the present invention is preferably between10-50, more preferably 11-45, even more preferably 12-45, most preferably 13-45, in particular 14-45, more particular 15-45, even more particular 16-40 amino acids long. Preferably the naturally occurring amino acid sequence which does not have the function of a signal peptide in nature of the present invention is of eukaryotic origin and not identical to any signal peptide of eukaryotic origin, more preferably is of mammalian origin and not identical to any signal peptide of mammalian origin, more preferably is of human origin and not identical to any signal peptide of human origin occurring in nature. A naturally occurring amino acid sequence which does not have the function of a signal peptide in nature is usually an amino acid sequence of the coding sequence of a protein. A naturally occurring amino acid sequence which does not have the function of a signal peptide in nature according to the present invention is usually of eukaryotic origin, preferably of mammalian origin, more preferably of human origin.

The term "naturally occurring", "natural" and "in nature" as used herein have the equivalent meaning.

The term "amino acids 1-9 of the N-terminal end of the amino acid sequence of the signal peptide" as used herein refers to the first nine amino acids of the N-terminal end of the amino acid sequence of a signal peptide.

The term "amino acid sequence modified by insertion, deletion and/or substitution of at least one amino acid" as used herein refers to an amino acid sequence which includes an amino acid substitution, insertion, and/or deletion of at least one amino acid within the amino acid sequence. The term "signal peptide heterologous to said protein is modified by insertion, deletion and/or substitution of at least one amino acid" as used herein refers to an amino acid sequence of a naturally occurring signal peptide heterologous to a protein which includes an amino acid substitution, insertion, and/or deletion of at least one amino acid within its naturally occurring amino acid sequence. The term "signal peptide homologous to said protein is modified by insertion, deletion and/or substitution of at least one amino acid" as used herein refers to a natural occurring signal peptide homologous to a protein which includes an amino acid substitution, insertion, and/or deletion of at least one amino acid within its naturally occurring amino acid sequence. The term "the naturally occurring amino acid sequence is modified by insertion, deletion and/or substitution of at least one amino acid" refers to a naturally occurring amino acid sequence which includes an amino acid substitution, insertion, and/or deletion of at least one amino acid within its naturally occurring amino acid sequence. By "amino acid substitution" or "substitution" herein is meant the replacement of an amino acid at a particular position in a parent protein sequence with another amino acid. For example, the substitution R34K refers to a polypeptide, in which the arginine at position 34 is replaced with a lysine. For the preceding example, 34K indicates the substitution of position 34 with a lysine. For the purposes herein, multiple substitutions are typically separated by a slash. For example, R34K/L78V refers to a double variant comprising the substitutions R34K and L38V. By "amino acid insertion" or "insertion" as used herein is meant the addition of an amino acid at a particular position in a parent protein sequence. For example, insert -34 designates an insertion at position 34. By "amino acid deletion" or "deletion" as used herein is meant the removal of an amino acid at a particular position in a parent protein sequence. For example, R34- designates the deletion of arginine at position 34.

The term" insulin-like growth factor 1", "insulin-like growth factor 1(IGF1)" or "IGF1" as used herein usually refers to the natural sequence of the IGF1 protein without the signalling peptide and may comprise the propeptide and/or the E-peptide and preferably refers to the natural sequence of the IGF1 protein without the signalling peptide and without the E-peptide. The term "human insulin-like growth factor 1(IGF1)" as used herein refers to the natural sequence of human IGF1 (pro-IGF1 which is referred to in the Uniprot database as UniProtKB - P05019 and in the Genbank database as NM_000618.4, NM_001111285.2 and NM_001111283.2, or a fragment thereof. The natural DNA sequence encoding human insulin-like growth factor 1 may be codon-optimized. The natural sequence of human IGF1 comprises or consists of the human signalling peptide having 21 amino acids (nucleotides 1-63), the human propeptide (also called pro-domain) having 27 amino acids (nucleotides 64-144), the mature human IGF1 having 70 amino acids (nucleotides 145-354) and the C-terminal domain of human IGF1 which is the so-called E-peptide (or E-domain). The C-terminal domain of human IGF1 (so called E-peptide or E-domain) comprises the Ea-, Eb- or Ec-domain which are generated by alternative splicing events. The Ea-domain comprises or consists of 35 amino acids (105 nucleotides), the Eb-domain comprises or consists of 77 amino acids (231 nucleotides), and the Ec-domain comprises or consists of 40 amino acids (120 nucleotides) (see e.g. Wallis M (2009) New insulin-like growth factor (IGF)-precursor sequences from mammalian genomes: the molecular evolution of IGFs and associated peptides in primates. Growth Horm IGF Res 19(1):12-23. doi: 10.1016/j.ghir.2008.05.001). The term "human insulin-like growth factor 1(IGF)"as used herein usually refers to the natural sequence of the human IGF1 protein without the signalling peptide and may comprise the propeptide and/or the E-peptide and preferably refers to the natural sequence of the human IGF1 protein without the signalling peptide and without the E-peptide. The term "human insulin-like growth factor 1(IGF)"as used herein usually comprises the mature human IGF1. The term "mature protein" refers to the protein synthesised in the endoplasmatic reticulum and secreted via the Golgi apparatus in a cell expressing and secreting the protein. The term "mature IGF1" refers to the protein synthesised in the endoplasmatic reticulum and secreted via the Golgi apparatus in a cell expressing and secreting IGF1. The term "mature human IGFI" refers to the protein synthesised in the endoplasmatic reticulum and secreted via the Golgi apparatus in a human cell expressing and secreting human IGF1 and normally contains the amino acids encoded by nucleotide sequence as shown in SEQ ID NO: 2

The term "the mRNA comprises a nucleic acid sequence encoding the propeptide of IGF1, and a nucleic acid sequence encoding the mature IGF1 and does not comprise a nucleic acid sequence encoding an E-peptide of IGF1" as used herein refers usually to a mRNA which comprises a nucleotide sequence encoding the propeptide (also called pro-domain) of human IGF1 having 27 amino acids, and a nucleotide sequence encoding the mature human IGF1 having 70 amino acids and which does not comprise a nucleotide sequence encoding an E-peptide (also called E-domain) of human IGF1 i.e. does not comprise a nucleotide sequence encoding a Ea-, Eb- or Ec-domain. The nucleotide sequence encoding the propeptide (also called pro-domain) of human IGF1 having 27 amino acids, and the nucleotide sequence encoding the mature human IGF1 having 70 amino acids may be codon optimized.

The term "vector" or "expression vector" as used herein refers to naturally occurring or synthetically generated constructs for uptake, proliferation, expression or transmission of nucleic acids in a cell, e.g. plasmids, minicircles, phagemids, cosmids, artificial chromosomes/mini-chromosomes, bacteriophages, viruses such as baculovirus, retrovirus, adenovirus, adeno-associated virus, herpes simplex virus, bacteriophages. Vectors can either integrate into the genome of the host cell or remain as autonomously replicating construct within the host cell. Methods used to construct vectors are well known to a person skilled in the art and described in various publications. In particular techniques for constructing suitable vectors, including a description of the functional and regulatory components such as promoters, enhancers, termination and polyadenylation signals, selection markers, origins of replication, and splicing signals, are known to the person skilled in the art. The eukaryotic expression vectors will typically contain also prokaryotic sequences that facilitate the propagation of the vector in bacteria such as an origin of replication and antibiotic resistance genes for selection in bacteria which might be removed before transfection of eukaryotic cells. A variety of eukaryotic expression vectors, containing a cloning site into which a polynucleotide can be operably linked, are well known in the art and some are commercially available from companies such as Agilent Technologies, Santa Clara, Calif.; Invitrogen, Carlsbad, Calif.; Promega, Madison, Wis. or Invivogen, San Diego, Calif.

The term "gene therapy vector" as used herein refers to any vector that is being used to deliver a nucleic acid sequence e.g. a nucleic acid sequence coding for a gene into cells. Gene therapy vectors and methods of gene delivery are well known in the art. Non-limiting examples of these methods include viral vector delivery systems including DNA and RNA viruses, which have either episomal or integrated genomes after delivery to the cell, non-viral vector delivery systems including DNA plasmids, naked nucleic acid, and nucleic acid complexed with a delivery vehicle, transposon system (for delivery and integration into the host genomes; Moriarity, et al. (2013) Nucleic Acids Res 41(8), e92, Aronovich, et al., (2011) Hum. Mol. Genet. 20(R1), R14-R20), retrovirus-mediated DNA transfer (e.g., Moloney Mouse Leukemia Virus, spleen necrosis virus, retroviruses such as Rous Sarcoma Virus, Harvey Sarcoma Virus, avian leukosis virus, gibbon ape leukemia virus, human immunodeficiency virus, adenovirus, Myeloproliferative Sarcoma Virus, and mammary tumor virus; see e.g., Kay et al. (1993) Science 262, 117-119, Anderson (1992) Science 256, 808-813), and DNA virus-mediated DNA transfer including adenovirus, herpes virus, parvovirus and adeno-associated virus (e.g., Ali et al. (1994) Gene Therapy 1, 367-384). Viral vectors also include but are not limited to adeno-associated virus, adenoviral virus, lentivirus, retroviral, and herpes simplex virus vectors. Vectors capable of integration in the host genome include but are not limited to retrovirus or lentivirus.

The term "transcription unit", "expression unit" or "expression cassette" as used herein refers a region within a vector, construct or polynucleotide sequence that contains one or more genes to be transcribed, wherein the genes contained within the segment are operably linked to each other. They are transcribed from a single promoter and transcription is terminated by at least one polyadenylation signal. As a result, the different genes are at least transcriptionally linked. More than one protein or product can be transcribed and expressed from each transcription unit (multicistronic transcription unit). Each transcription unit will comprise the regulatory elements necessary for the transcription and translation of any of the selected sequence that are contained within the unit and each transcription unit may contain the same or different regulatory elements. For example, each transcription unit may contain the same terminator. IRES element or introns may be used for the functional linking of the genes within a transcription unit. A vector or polynucleotide sequence may contain more than one transcription unit.

The term "smooth muscle-related diseases" as used herein refers to diseases or conditions such as stress urinary incontinence (SUI), mixed urinary incontinence, urge urinary incontinence, under active bladder, and overflow incontinence.

The term "lower urinary tract symptoms" abbreviated as "LUTS" as used herein refers to frequency, nocturia, urgency, incomplete voiding and urinary incontinence and comprises diseases or conditions such as stress urinary incontinence (SUI), mixed urinary incontinence, urge urinary incontinence, under active bladder, overflow incontinence and pelvic organ prolapse.

The term "stress urinary incontinence", also known as stress incontinence or effort incontinence and its abbreviation "SUI" which is used synonymously herein refers to the loss of urine associated with coughing, laughing, sneezing, exercising or other movements that increase intra-abdominal pressure and thus increasing the pressure on the bladder. It is due to inadequate closure of the bladder outlet by the external (striated muscle) and internal (smooth muscle) urethral sphincters. If this support is insufficient due to any reason, the urethra cannot close properly at times of increased abdominal pressure, allowing urine to pass involuntarily. Stress urinary incontinence is the most preferred lower urinary tract symptom to be treated with the method of the present invention.

The term "mixed urinary incontinence", also known as "mixed incontinence" which is used synonymously herein refers to a combination of stress and urge incontinence, and shares symptoms of both. Where urge incontinence is caused by involuntary actions of the bladder muscles. These may occur because of damage to nerves of the bladder, the nervous system, or muscles themselves. Mixed urinary incontinence is the second most preferred lower urinary tract symptom to be treated with the method of the present invention.

The term "urge urinary incontinence", as used herein refers to the loss of urine due to abnormal bladder contractions overriding the sphincter muscles of the urethra. These may occur as a results of central nervous system disorders (such as Alzheimer's disease, multiple sclerosis, and Parkinson's disease), interstitial cystitis, urinary tract infection, or pelvic radiation. Urge urinary incontinence is the third most preferred lower urinary tract symptom to be treated with the method of the present invention.

The term "under active bladder", also known as bladder underactivity and detrusor (smooth) muscle underactivity and the abbreviation UAB which is used synonymously herein refers to difficulty with bladder emptying, such as hesitancy to start the stream, a poor or intermittent stream, or sensations of incomplete bladder emptying. Under active bladder is the fourth most preferred lower urinary tract symptom to be treated with the method of the present invention.

The term "overflow incontinence", as used herein refers to a form of urinary incontinence, characterized by the involuntary release of urine from an overfull urinary bladder, often in the absence of any urge to urinate. This condition occurs in people who have a blockage of the bladder outlet, or when the destrusor (smooth) muscle that expels urine from the bladder is too weak to empty the bladder normally. Overflow incontinence is the fith most preferred lower urinary tract symptom to be treated with the method of the present invention.

The term "pelvic organ prolapse" abbreviated as "POP") as used herein refers to a lower urinary tract symptom which is characterized by descent of pelvic organs from their normal positions. In women, the condition usually occurs when the pelvic floor collapses after gynecological cancer treatment, childbirth or heavy lifting. A POP occurs when the muscles, fascia, tendons and connective tissues of the pelvic floor (striated) muscles weaken. POP is the sixth most preferred lower urinary tract symptom to be treated with the method of the present invention.

In a first aspect the present invention provides a mRNA or a therapeutic composition thereof for use in a method of treating lower urinary tract symptoms (LUTS), wherein the mRNA comprises a nucleic acid sequence encoding insulin-like growth factor 1 (IGF1). Also disclosed herein is a mRNA or a therapeutic composition thereof as described herein for use in a method of treating smooth muscle-related diseases.

In one embodiment of the invention the mRNA or the therapeutic composition thereof for use in a method of treating LUTS is such that the mRNA comprises a nucleic acid sequence encoding a signal peptide, optionally a nucleic acid sequence encoding the propeptide of IGF1 and a nucleic acid sequence encoding the mature IGF1. Preferably the nucleic acid sequence encoding the signal peptide encodes the signal peptide of the brain-derived neurotrophic factor (BDNF).

In another embodiment of the invention, the mRNA or the therapeutic composition thereof for use in a method of treating LUTS according to the invention is such that the mRNA comprises a nucleic acid sequence encoding the signal peptide of the brain-derived neurotrophic factor (BDNF), a nucleic acid sequence encoding the propeptide of human IGF1, a nucleic acid sequence encoding the mature human IGF1 and does not comprise a nucleic acid sequence encoding an E-peptide of human IGF1.

In a preferred embodiment the present invention provides the abovementioned mRNA or a therapeutic composition thereof for use in a method of treating lower urinary tract symptoms, wherein the lower urinary tract symptoms are selected from the group consisting of stress urinary incontinence (SUI), mixed urinary incontinence, urge urinary incontinence, under active bladder, overflow incontinence and pelvic organ prolapse. In a more preferred embodiment the present invention provides the abovementioned mRNA or a therapeutic composition thereof for use in a method of treating lower urinary tract symptoms, wherein the lower urinary tract symptoms are selected from the group consisting of stress urinary incontinence (SUI), mixed urinary incontinence, urge urinary incontinence, under active bladder and overflow incontinence.

In an even more preferred embodiment the present invention provides the abovementioned mRNA or a therapeutic composition thereof for use in a method of treating lower urinary tract symptoms, wherein the lower urinary tract symptoms are selected from the group consisting of stress urinary incontinence (SUI), mixed urinary incontinence, urge urinary incontinence and under active bladder.

In a particular preferred embodiment the present invention provides the abovementioned mRNA or a therapeutic composition thereof for use in a method of treating lower urinary tract symptoms, wherein the lower urinary tract symptoms are selected from the group consisting of stress urinary incontinence (SUI), mixed urinary incontinence and urge urinary incontinence.

In a more particular preferred embodiment the present invention provides the abovementioned mRNA or a therapeutic composition thereof for use in a method of treating lower urinary tract symptoms, wherein the lower urinary tract symptoms are selected from the group consisting of stress urinary incontinence (SUI) and mixed urinary incontinence.

In an even more particular preferred embodiment the present invention provides the abovementioned mRNA or a therapeutic composition thereof for use in a method of treating stress urinary incontinence (SUI).

Also disclosed is an embodiment wherein the mRNA comprises a nucleic acid sequence encoding a protein, wherein the protein is a growth factor and a nucleic acid sequence encoding a signal peptide. Thus in one also disclosed embodiment the mRNA comprises a nucleic acid sequence encoding a signal peptide and a nucleic acid sequence encoding a growth factor. In a further also disclosed embodiment the signal peptide of the brain-derived neurotrophic factor (BDNF) replaces the natural signal peptide of the protein
In one also disclosed embodiment the mRNA comprises a nucleic acid sequence encoding a signal peptide and a nucleic acid sequence encoding encoding insulin-like growth factor 1 (IGF1).

In another also disclosed embodiment the growth factor is selected from the group consisting of EGF, FGF1, GDNF, IGF1, IGF2, NTF3, TGFB1, more preferably selected from the group consisting of IGF1 and IGF2. Most particular, the protein is IGF1, preferably human IGF1.

In some further also disclosed embodiments the mRNA is a mRNA comprising a nucleic acid sequence encoding a growth factor and a nucleic acid sequence encoding a signal peptide, wherein the amino acids 1-9 of the N-terminal end of the amino acid sequence of the signal peptide have an average hydrophobic score of above 2.

In some further also disclosed embodiments the mRNA is a mRNA comprising a nucleic acid sequence encoding a growth factor and a nucleic acid sequence encoding a signal peptide, wherein the amino acids 1-9 of the N-terminal end of the amino acid sequence of the signal peptide have an average hydrophobic score of above 2, wherein the signal peptide is selected from the group consisting of
i) a signal peptide heterologous to said protein, wherein the signal peptide heterologous to said protein is optionally modified by insertion, deletion and/or substitution of at least one amino acid;
ii) a signal peptide homologous to said protein, wherein the signal peptide homologous to said protein is modified by insertion, deletion and/or substitution of at least one amino acid; and
iii) a naturally occurring amino acid sequence which does not have the function of a signal peptide in nature, wherein the naturally occurring amino acid sequence is optionally modified by insertion, deletion and/or substitution of at least one amino acid.

Preferably the deleted amino acid is an amino acid with a hydrophobic score of below -0.8, preferably below 1.9. Preferably the substitute amino acid is an amino acid with a hydrophobic score which is higher than the hydrophobic score of the substituted amino acid, more preferably the substitute amino acid is an amino acid with a hydrophobic score of 2.8 and higher, or more preferably with a hydrophobic score of 3.8 and higher. Preferably the inserted amino acid is an amino acid with a hydrophobic score of 2.8 and higher, or more preferably with a hydrophobic score of 3.8 and higher.

Usually between 1 and 15, preferably between 1 and 11 amino acids, more preferably between 1 and 10 amino acids, even more preferably between 1 and 9 amino acids, in particular between 1 and 8 amino acids, more particular between 1 and 7 amino acids, even more particular between 1 and 6 amino acids, particular preferably between 1 and 5 amino acids, more particular preferably between 1 and 4 amino acids, or even more particular preferably between 1 and 2 amino acids in a given amino acid sequence are inserted, deleted, and/or substituted. Usually between 1 and 15, preferably between 1 and 11 amino acids, more preferably between 1 and 10 amino acids, even more preferably between 1 and 9 amino acids, in particular between 1 and 8 amino acids, more particular between 1 and 7 amino acids, even more particular between 1 and 6 amino acids, particular preferably between 1 and 5 amino acids, more particular preferably between 1 and 4 amino acids, or even more particular preferably between 1 and 2 amino acids in a given amino acid sequence are inserted, deleted, and/or substituted usually within the amino acids 1-11, preferably within the amino acids 1-10, more preferably within the amino acids 1-9, even more preferably within the amino acids 1-8, in particular within the amino acids 1-7, more particular within the amino acids 1-6, even more particular within the amino acids 1-5, particular preferably within the amino acids 1-4, more particular preferably within the amino acids 1-3, or even more particular preferably within the amino acids 1-2 of the N-terminal end of the amino acid sequence of the target motif or the signal peptide. Preferably the amino acid sequence is optionally modified by deletion, and/or substitution of at least one amino acid.

The term "hydrophobic score" or "hydrophobicity score" is used synonymously to the term "hydropathy score" herein and refers to the degree of hydrophobicity of an amino acid as calculated according to the Kyte-Doolittle scale (Kyte J., Doolittle R.F.; J. Mol. Biol. 157:105-132(1982)). The amino acid hydrophobic scores according to the Kyte-Doolittle scale are as follows:

| **Amino** Acid | **One Letter Code** | **Hydrophobic Score** |
|---|---|---|
| Isoleucine | I | 4.5 |
| Valine | V | 4.2 |
| Leucine | L | 3.8 |
| Phenylalanine | F | 2.8 |
| Cysteine | C | 2.5 |
| Methionine | M | 1.9 |
| Alanine | A | 1.8 |
| Glycine | G | -0.4 |
| Threonine | T | -0.7 |
| Serine | S | -0.8 |
| Tryptophan | W | -0.9 |
| Tyrosine | Y | -1.3 |
| Proline | P | -1.6 |
| Histidine | H | -3.2 |
| Glutamic acid | E | -3.5 |
| Glutamine | Q | -3.5 |
| Aspartic acid | D | -3.5 |
| Asparagine | N | -3.5 |
| Lysine | K | -3.9 |
| Arginine | R | -4.5 |

The "average hydrophobic score" of an amino acid sequence e.g. the average hydrophobic score of the amino acids 1-9 of the N-terminal end of the amino acid sequence of a signal peptide is calculated by adding the hydrophobic score according to the Kyte-Doolittle scale of each of the amino acid of the amino acid sequence e.g. the hydrophobic score of each of the nine amino acids of the amino acids 1-9 of the N-terminal end, divided by the number of the amino acids, e.g divided by nine.

In one embodiment of the present invention the amino acids 1-9 of the N-terminal end of the amino acid sequence of the signal peptide have an average hydrophobic score of equal to or above 2.05, preferably of equal to or above 2.1, more preferably of equal to or above 2.15, even more preferably of equal to or above 2.2, in particular of equal to or above 2.25, more particular of equal to or above 2.3, even more particular of equal to or above 2.35. In a further embodiment the amino acids 1-9 of the N-terminal end of the amino acid sequence of the signal peptide have an average hydrophobic score of between 2.05 and 4.5, preferably between 2.1 and 4.5, more preferably between 2.15 and 4.5, even more preferably between 2.2 and 4.5, in particular between 2.25 and 4.5, more particular between 2.3 and 4.5, even more particular between 2.35 and 4.5. In a further embodiment the amino acids 1-9 of the N-terminal end of the amino acid sequence of the signal peptide have an average hydrophobic score of between 2.05 and 4.0, preferably between 2.1 and 4.0, more preferably between 2.15 and 4.0, even more preferably between 2.2 and 4.0, in particular between 2.25 and 4.0, more particular between 2.3 and 4.0, even more particular between 2.35 and 4.0.

In one embodiment of the present invention the amino acids 1-9 of the N-terminal end of the amino acid sequence of the signal peptide have an average polarity of 6.1 or below, preferably an average polarity of below 6.1, more preferably an average polarity of below 4, even more preferably an average polarity of below 2, in particular an average polarity of between 6.1 and 0, more particular an average polarity of between 4 and 0, even more particular an average polarity of between 2 and 0., most particular an average polarity of between 1 and 0.2. Preferably, the average hydrophobic score of the first nine amino acids of the N-terminal end of the amino acid sequence of the modified signal peptide is increased 1.0 unit or above compared to the signal peptide without modification.

The polarity is calculated according to Zimmerman Polarity index (Zimmerman J.M., Eliezer N., Simha R.; J. Theor. Biol. 21:170-201(1968)). The "average polarity" of an amino acid sequence e.g. the average polarity of the amino acids 1-9 of the N-terminal end of the amino acid sequence of a signal peptide is calculated by adding the polarity value calculated according to Zimmerman Polarity index of each of the amino acid of the amino acid sequence e.g. the average polarity of each of the nine amino acids of the amino acids 1-9 of the N-terminal end, divided by the number of the amino acids, e.g divided by nine. The polarity of amino acids according to Zimmerman Polarity index is as follows:

| **Amino** Acid | One Letter Code | **Polarity** |
|---|---|---|
| Isoleucine | I | 0.13 |
| Valine | V | 0.13 |
| Leucine | L | 0.13 |
| Phenylalanine | F | 0.35 |
| Cysteine | C | 1.48 |
| Methionine | M | 1.43 |
| Alanine | A | 0 |
| Glycine | G | 0 |
| Threonine | T | 1.66 |
| Serine | S | 1.67 |
| Tryptophan | W | 2.1 |
| Tyrosine | Y | 1.61 |
| Proline | P | 1.58 |
| Histidine | H | 51.6 |
| Glutamic acid | E | 49.9 |
| Glutamine | Q | 3.53 |
| Aspartic acid | D | 49.7 |
| Asparagine | N | 3.38 |
| Lysine | K | 49.5 |
| Arginine | R | 52 |

The above mentioned average hydrophobic score or average polarity of an amino acid sequence of a signal peptide of the present invention can be calculated by using the publicly available online database **ProtScale** (http://www.expasy.org/tools/protscale.html) referred to in Gasteiger E. et al. (Gasteiger E., Hoogland C., Gattiker A., Duvaud S., Wilkins M.R., Appel R.D., Bairoch A.;Protein Identification and Analysis Tools on the ExPASy Server;(In) John M. Walker (ed): The Proteomics Protocols Handbook, Humana Press (2005).pp. 571-607) with the selection of Hydrophobicity of Kyte & Doolittle scale ("Hphob. / Kyte & Doolittle") or polarity of Zimmerman scale ("Polarity / Zimmerman") and settings corresponding to a specific window size (e.g. window size of 9 amino acids) of a signal peptide, with the window edge relative weight value set to 100%, and without scale normalization. The respective numerical value data can be retrieved by opening link on 'Numerical format (verbose)' in the result page.

In a preferred embodiment of the present invention the signal peptide is the signal peptide of brain-derived neurotrophic factor (BDNF), more preferably the signal peptide of human brain-derived neurotrophic factor (BDNF), even more preferably the signal peptide as shown in SEQ ID NO: 3, in particular the signal peptide of the human BDNF encoded by the nucleic acid sequence as shown in SEQ ID NO: 4
Also disclosed is a mRNA or a therapeutic composition thereof for use in a method of treating lower urinary tract symptoms, the mRNA comprising a nucleic acid sequence encoding a growth factor and a nucleic acid sequence encoding the signal peptide of the brain-derived neurotrophic factor (BDNF).

In one embodiment of the present invention the mRNA is naked mRNA. In a preferred embodiment, the mRNA comprises an antireverse CAP analog such as m7G(5')G, m7GpppG cap, an internal ribosome entry site (IRES) and/or a polyA tail at the 3' end in particular in order to improve translation. The mRNA can have further regions promoting translation known to the skilled person.

In a preferred embodiment of the present invention the mRNA contains a combination of modified and unmodified nucleotides. In a more preferred embodiment, in such a modified mRNA 1 to 100%, preferably 10 to 100%, more preferably 50 to 100%, even more preferably 90 to 100%, most preferably 100% of the uridine nucleotides are modified. The adenosine-, guanosine-, and cytidine-containing nucleotides can be unmodified or partially modified, and they are preferably present in unmodified form. Preferably the content of the modified uridine nucleotides in the mRNA lies in a range from 5 to 25%. In a particularly preferred embodiment of the present inventionthe modified uridine nucleotides are N¹-Methylpseudouridines. In a more particularly preferred embodiment of the present invention the mRNA contains a combination of modified and unmodified nucleotides, wherein in such a modified mRNA 1 to 100%, preferably 10 to 100%, more preferably 50 to 100%, even more preferably 90 to 100%, most preferably 100% of the uridine nucleotides are N¹-Methylpseudouridines.

In a more preferred embodiment of the present invention the mRNA is an mRNA which is codon optimized and contains a combination of modified and unmodified nucleotides. In a more preferred embodiment, in such a modified mRNA 1 to 100%, preferably 10 to 100%, more preferably 50 to 100%, even more preferably 90 to 100%, most preferably 100% of the uridine nucleotides are modified. The adenosine-, guanosine-, and cytidine-containing nucleotides can be unmodified or partially modified, and they are preferably present in unmodified form. Preferably the content of the modified uridine nucleotides in the mRNA lies in a range from 5 to 25%. In a particularly preferred embodiment of the present invention the modified uridine nucleotides are N¹-Methylpseudouridines. In a more particularly preferred embodiment of the present invention the RNA is mRNA which contains a combination of modified and unmodified nucleotides, wherein in such a modified mRNA 1 to 100%, preferably 10 to 100%, more preferably 50 to 100%, even more preferably 90 to 100%, most preferably 100% of the uridine nucleotides are N¹-Methylpseudouridines.

In a preferred embodiment of the present invention the mRNA comprises a nucleic acid sequence encoding human insulin-like growth factor 1 (IGF1) as protein, more preferably the mRNA is naked mRNA comprising a nucleic acid sequence encoding human insulin-like growth factor 1 (IGF1) as protein. In this preferred embodiment of the present invention the mRNA comprises a nucleic acid sequence encoding the mature human IGF-1.

In a more preferred embodiment of the present invention the mRNA comprises a nucleic acid sequence encoding the propeptide of IGF1, preferably the propeptide of human IGF1, and a nucleic acid sequence encoding the mature protein of IGF1, preferably the mature protein of human IGF1, and does not comprise a nucleic acid sequence encoding an E-peptide of IGF1, preferably does not comprise a nucleic acid sequence encoding a E-peptide of human IGF1. In a further more preferred embodiment of the present invention the mRNA comprises a nucleic acid sequence encoding the propeptide of IGF1, preferably the propeptide of human IGF1, a nucleic acid sequence encoding the mature protein of IGF1, preferably the mature protein of human IGF1. Preferably the mRNA does not comprise a nucleic acid sequence encoding an E-peptide of IGF1, more preferably does not comprise a nucleic acid sequence encoding a E-peptide of human IGF1. In a further more preferred embodiment of the present invention the mRNA comprises a nucleic acid sequence encoding the propeptide of IGF1, preferably the propeptide of human IGF1, a nucleic acid sequence encoding the mature protein of IGF1, preferably the mature protein of human IGF1 and a nucleic acid sequence encoding the signal peptide of the brain-derived neurotrophic factor (BDNF) . Preferably the mRNA does not comprise a nucleic acid sequence encoding an E-peptide of IGF1, more preferably does not comprise a nucleic acid sequence encoding a E-peptide of human IGF1.

In an even more preferred embodiment of the present invention the mRNA comprises a nucleotide acid sequence encoding the propeptide (also called pro-domain) of IGF1, preferably of human IGF1 having 27 amino acids, and a nucleotide sequence encoding the mature IGF1, preferably the mature human IGF1 having 70 amino acids, and preferably does not comprise a nucleotide sequence encoding an E-peptide of IGF1, preferably does not comprise a nucleic acid sequence encoding a E-peptide of human IGF1.

In a further even more preferred embodiment of the present invention the mRNA comprises a nucleotide acid sequence encoding the propeptide (also called pro-domain) of IGF1, preferably of human IGF1 having 27 amino acids, a nucleotide sequence encoding the mature IGF1, preferably the mature human IGF1 having 70 amino acids and a nucleic acid sequence encoding the signal peptide of the brain-derived neurotrophic factor (BDNF). Preferably the mRNA does not comprise a nucleotide sequence encoding an E-peptide of IGF1, more preferably does not comprise a nucleic acid sequence encoding a E-peptide of human IGF1.

In a particular preferred embodiment of the present invention the mRNA comprises a nucleic acid sequence encoding the propeptide (also called pro-domain) of human IGF1 having 27 amino acids, and a nucleotide acid sequence encoding the mature human IGF1 having 70 amino acids and preferably does not comprise a nucleotide sequence encoding an E-peptide (also called E-domain) of human IGF1, wherein the nucleotide sequence encoding the propeptide (also called pro-domain) of human IGF1 having 27 amino acids, and the nucleotide sequence encoding the mature human IGF1 having 70 amino acids and the nucleotide sequence encoding the E-peptides are as referred to in the Uniprot database as UniProtKB - P05019 and in the Genbank database as NM_000618.4, NM_001111285.2 and NM_001111283.2, respectively.

In an even more particular preferred embodiment of the present invention the mRNA comprises a nucleic acid sequence encoding the propeptide (also called pro-domain) of human IGF1 having 27 amino acids as shown in SEQ ID NO: 1 and a nucleotide acid sequence encoding the mature human IGF1 having 70 amino acids as shown in SEQ ID NO: 2, and preferably does not comprise a nucleotide sequence encoding an E-peptide (also called E-domain) of human IGF1.

In a further even more particular preferred embodiment of the present invention the mRNA comprises a nucleic acid sequence encoding the propeptide (also called pro-domain) of human IGF1 having 27 amino acids as shown in SEQ ID NO: 1, a nucleotide acid sequence encoding the mature human IGF1 having 70 amino acids as shown in SEQ ID NO: 2. and a nucleic acid sequence encoding the signal peptide of the brain-derived neurotrophic factor (BDNF), preferably a nucleotide acid sequence encoding the signal peptide of the brain-derived neurotrophic factor (BDNF) as shown in SEQ ID NO: 4. Preferably the mRNA does not comprise a nucleotide sequence encoding an E-peptide (also called E-domain) of human IGF1.

In a particular preferred embodiment of the present invention the mRNA or the therapeutic composition thereof for use in a method of treating LUTS comprises a nucleic acid sequence as shown in SEQ ID NO: 8.

In a further particular preferred embodiment of the present invention the mRNA comprises a nucleic acid sequence transcribed from the DNA sequence as shown in SEQ ID NO: 7. Preferably the nucleic acid sequence is transcribed from the DNA sequence as shown in SEQ ID NO: 7 in vitro.

In a more particular preferred embodiment of the present invention the mRNA comprises a nucleic acid sequence as shown in SEQ ID NO: 8 wherein preferably 1 to 100%, more preferably 50 to 100%, even more preferably 90 to 100%, most preferably 100% of the uridine nucleotides are N¹-Methylpseudouridines.

In a further more particular preferred embodiment of the present invention the mRNA comprises a nucleic acid sequence transcribed from the DNA sequence as shown in SEQ ID NO: 7, wherein preferably 1 to 100%, more preferably 50 to 100%, even more preferably 90 to 100%, most preferably 100% of the uridine nucleotides are N¹-Methylpseudouridines. In this embodiment the nucleotide sequence is preferably transcribed from the DNA sequence as shown in SEQ ID NO: 7 in vitro, whereas as uridine nucleotides only N¹-Methylpseudouridine-5'-Triphosphate (N¹-Methylpseudo-UTP) i.e. 100% N¹-Methylpseudo-UTP is used for the transcription from the DNA sequence as shown in SEQ ID NO: 7

In a more preferred embodiment of the present invention the mRNA comprises a nucleic acid sequence encoding in the following order from 5' to 3' :
i) the signal peptide of the brain-derived neurotrophic factor (BDNF);
ii) optionally a pro-domain of a growth factor; and
iii) the mature growth factor protein.

In an even more preferred embodiment of the present invention the mRNA comprises a nucleic acid sequence encoding in the following order from 5' to 3':
i) the signal peptide of the brain-derived neurotrophic factor (BDNF);
ii) optionally a pro-domain of IGF1; and
iii) the mature human IGF1.

In a particular preferred embodiment of the present invention the mRNA comprises a nucleic acid sequence encoding in the following order from 5' to 3':
i) the signal peptide of the brain-derived neurotrophic factor (BDNF);
ii) optionally a pro-domain of human IGF1; and
iii) the mature human IGF1.

Also disclosed herein is a transcription unit, an expression vector or a gene therapy vector comprising the nucleic acid sequence of the mRNA as described above or the nucleic acid sequence of a DNA from which the mRNA as described above has been transcribed.

Usually the mRNA of the present invention is provided as therapeutic composition, which is preferably a liquid composition. A liquid composition is any composition in which the mRNA is present in solution in a liquid. In one embodiment of the present invention the mRNA is solved in water, or a buffered or unbuffered aqueous solution. The solution is preferably an aqueous solution. Thus, the liquid may be water, preferably sterile water, more preferably "water for injection" (WFI) or any other buffered or unbuffered aqueous solution. In one embodiment of the present invention the liquid composition is an unbuffered solution, preferably a salt solution, more preferably a salt solution of a pharmaceutically acceptable salt, even more preferably a NaCl solution, i.e. saline. Preferably, the salt solution is isotonic and even more preferably it shows a physiological pH value. In a preferred embodiment of the present invention the solution in which the mRNA is contained is a buffered solution. Preferably, such a solution is isotonic to blood. In principle any buffer which effectively buffers in the physiological range, in particular in the range of pH 3.0 to 10.5 and more preferably pH 4.0 to 9.0, can be used. Preferable buffers are acetate, phosphate, phosphate buffered saline (PBS), carbonate, lactate and citrate buffers or Ringer's solution, preferably citrate buffer. Thus in a more preferred embodiment of the present invention the solution in which the mRNA is contained is citrate buffer.

The concentration of the mRNA in the therapeutic composition is not particularly crucial and can be adjusted as required. Preferably, the concentration lies in the range of 0.001 to 20.0 µg/µl, more preferably in the range of 0.01 to 10.0 µg/µl, even more preferably in the range of 0.1 to 5 µg/l, in particular in the range of 0.4 to 2.0 µg/µl, more particular in the range of 0.6 to 1.5 µg/µl, even more particular in the range of 0.80 to 1.20 µg/µl. Particular preferred is a range of 0.01 µg to 0.1 g, preferably of 0.1 µg to 0.01 g, more preferably of 0.5 µg to 1 mg, even more preferably of 0.5 µg to 10 µg.

The mRNA and/or the therapeutic composition can be applied to cells and tissues e.g. urethral sphincter muscles by means known to the person skilled in the art, preferably by injection, more preferably by urethral intra-muscular injection, typically by using a syringe with a needle. In principle any commercially available syringe in combination with a needle, a guidance device and a commercially available cystoscope can be used for this purpose.. The diameter of a needle is indicated by the needle gauge (G; according to the Stub's Needle Gauge). Typically needles in medical use range from 7 G (the largest) to 33 G (the smallest) can be used.

In some embodiments, the mRNA and/or the therapeutic composition can be delivered to a cell via direct DNA transfer (Wolff et al. (1990) Science 247, 1465-1468). The mRNA and/or the therapeutic composition can be delivered to cells following mild mechanical disruption of the cell membrane, temporarily permeabilizing the cells. Such a mild mechanical disruption of the membrane can be accomplished by gently forcing cells through a small aperture (Sharei et al. PLOS ONE (2015) 10(4), e0118803). In another embodiment, the mRNA and/or the therapeutic composition can be delivered to a cell via liposome-mediated DNA transfer (e.g., Gao & Huang (1991) Biochem. Ciophys. Res. Comm. 179, 280-285, Crystal (1995) Nature Med. 1, 15-17, Caplen et al. (1995) Nature Med. 3, 39-46). The term "liposome" can encompass a variety of single and multilamellar lipid vehicles formed by the generation of enclosed lipid bilayers or aggregates. The mRNA can be encapsulated in the aqueous interior of a liposome, interspersed within the lipid bilayer of a liposome, attached to a liposome via a linking molecule that is associated with both the liposome and the oligonucleotide, entrapped in a liposome, or complexed with a liposome.

In one embodiment of the present invention the RNA or the therapeutic composition thereof is administered directly into the urethral sphincter muscle (preferably by injection) in the form of a therapeutic i.e. a liquid composition wherein the RNA is contained as naked RNA. As regards the way of administration and the characteristics of the composition and the RNA contained therein, the same applies as has been set forth herein elsewhere. In a preferred embodiment, the liquid composition and mRNA, respectively, of the present invention is to be administered directly into the urethral sphincter muscle. In this context, the most preferred way of administration is injection, i.e. intra-muscular injection.

It is, in principle, envisaged in the context of the invention to administer the mRNA and the therapeutic composition, respectively, as early as possible, i.e. at the earliest possible stage of the lower urinary tract symptoms. For example, this stage is once (a) first symptom(s) have/has been observed (e.g. loss of urine). However, any possible point of time after the diagnosis is possible and worthwhile and, hence, envisaged in accordance with the invention. In one embodiment, the mRNA and the therapeutic composition, respectively, is to be administered during or even before the inflammatory and early proliferative phase, respectively, of urethral sphincter muscle regeneration. In a preferred embodiment, the therapeutic composition is to be administered before the inflammatory phase which follows the said lower urinary tract symptoms.

The administration of the mRNA and the therapeutic composition, respectively, in accordance with the invention may, for example depending on the course of the injury to be treated, be repeated at least once but preferably several times (for example 3 to 5 times). The repeated administration may be after 1, 2, 3, 4 , 5 , 6, 7, 8, or 9 days, preferably after day 2, 3, 4, 5, 6, 7, more preferably after day 3, 4 or 5. The repeated administration may be every few weeks (for example every 1, 2, 3, or 4 weeks) up to every few days (for example every 1, 2, 3, 4, 5 or 6 days), preferably every 2 or 3 days.

The mRNA or the therapeutic composition of the invention can be administered to a patient at a suitable dose. The dosage regimen can be determined by the attending physician, for example based on clinical factors. As is well known in the medical arts, dosages for any one patient depend upon many factors, including the patient's size, body surface area, age, the particular compound to be administered, sex, time and route of administration, general health, and other drugs being administered concurrently. Corresponding samples may be taken from, for example, urethral sphincter muscle (e.g. by a suitable probe) and the active compounds (naked RNA) may be detected and their corresponding concentrations may be determined in said samples, for example by PCR.

A typical dose of active substances (e.g. mRNA) can be, for example, in the range of 1 ng to several grams, preferably in the range of 0.1 µg to 1 g, preferably in the range of 1 µg to 0.1 g, more preferably in the range of 3 µg to 1 mg, even more preferably in the range of 5 µg to 0.5 mg and most preferably in the range of 10 µg to 100 µg. Particular preferred is a range of 0.01 µg to 0.1 g, preferably of 0.1 µg to 0.01 g, more preferably of 0.5 µg to 1 mg, even more preferably of 0.5 µg to 50 µg. This particularly applies to a human patient. Applied to mRNA therapy, the dosage of an mRNA for expression should correspond to this range; however, doses below or above this exemplary range are, in principle, also envisioned, especially considering the aforementioned factors. Generally, the regimen as a regular administration of the therapeutic composition should be in the range of 0.1 µg to 10 mg units, preferably in the range of 1 µg to 1 mg units, more preferably in the range of 10 µg to 0.1 mg units per kilogram of body weight per day. Again, this is particularly applied to a human patient. Progress can be monitored by periodic assessment. Dosages may vary but a preferred dosage for administration by injection of mRNAs as constituents of the liquid composition of the present invention is from approximately 10⁵ to 10¹⁵ copies of the mRNA molecule per injection. Again, this particularly applies to a human patient.

In particular, the therapeutic composition of the invention is envisaged to be administered to a patient, preferably to a human patient/a human. However, the herein described lower urinary tract symptoms may also be treated (or prevented) in a non-human animal subject/patient like, for example, a pet (e.g. dog, cat, rabbit, rat and mouse), a cattle (e.g. cow, pig, sheep), a horse (e.g. a race horse) or pony, a camel (e.g. a race camel) or a bird (e.g. chicken, turkey, parrot).

In particular, the therapeutic composition comprising mRNA is therapeutically active in the healing process of LUTS.

In one embodiment the therapeutic effect of the mRNA administered in a rodent lasts for nine days or more after administration of the mRNA. It is known that physiological processes in human are much slower e.g. by a factor in the range of 50 to 300 than in rodents as can be seen e.g. from the scientific references Zuideveld et al., Pharmaceutical Research, Vol. 24, No. 11, 2007, 2031-2039 and Adolph, Science, Vol. 109, 1949, 579-585. Without being bound by theory, it is assumed that the long-lasting therapeutic effect of the mRNA of the present invention in rodents of nine days or more will last in human which have been administered with the mRNA of the present invention for at least three to six months. Thus in a further embodiment the therapeutic effect of the mRNA administered in a human lasts for at least three months after administration of the mRNA.

In one embodiment the concentration of the mRNA in the urethral sphincter muscle is by a factor of ten lower three days after administration in a rodent than the concentration of the mRNA in the urethral sphincter muscle when administered, wherein the therapeutic effect of the mRNA lasts for nine days or more.

In one embodiment the therapeutic effect is assessed by measuring the urinary bladder leak point pressure, wherein a ratio of the urinary bladder leak point pressure measured nine days after administration of the mRNA and of the urinary bladder leak point pressure measured when the mRNA is administered of at least 1.1 indicates a therapeutic effect.

Any of the therapeutic compositions of the invention may be provided together with an instruction manual or instruction leaflet. The instruction manual/leaflet may comprise guidance for the skilled person/attending physician how to treat (or prevent) a disease or disorder as described herein (lower urinary tract symptoms) in accordance with the invention.

In particular, the instruction manual/leaflet may comprise guidance as to the herein described mode of delivery/administration and delivery/administration regimen, respectively (for example route of delivery/administration, dosage regimen, time of delivery/administration, frequency of delivery/administration). In particular, the instruction manual/leaflet may comprise the instruction that the mRNA, respectively, is to be injected and/or is prepared for injection into urethral sphincter muscle. The instruction manual/leaflet may further comprise the instruction that the mRNA, respectively, is prepared for administration during the inflammatory phase which follows the lower urinary tract symptoms. In principle, what has been said herein elsewhere with respect to the mode of delivery/administration and delivery/administration regimen, respectively, may be comprised as respective instructions in the instruction manual/leaflet.

Also disclosed is a kit for use in a method of treating lower urinary tract symptoms comprising the mRNA and/or the transcription unit, the expression vector or the gene therapy vector or the therapeutic composition as described above, and instructions, optionally a vector map, optionally a host cell, optionally a cultivation medium for the cultivation of a host cell, and/or optionally a selection medium for selecting and cultivating a transfected host cell. The kit may be provided in (or in form of) a kit of contents. The kit may further comprise one or more of the components of the therapeutic composition of the invention, for example in one or more separate containers. For example, the kit may comprise the mRNA (e.g. in dried form), a solubilizer and (buffered or unbuffered) aqueous solution, for example in one, two or three (or more) separate containers, respectively. The kit may also comprise the instruction manual or instruction leaflet.

### Examples

These examples are provided for illustrative purposes only and not to limit the scope of the claims provided herein.

### METHODS AND MATERIAL

### Example 1: Construct design, sequence, and synthesis

IGF1 (UniProt # P05019) is a polypeptide synthesized in the endoplasmic reticulum and secreted via the Golgi apparatus to act as extracellular growth factor in an auto- and paracrine manner. For ensuring proper expression and secretion of mRNA-induced IGF1 out of the transfected cell, the endogenous IGF1 pre-domain (signal peptide; SEQ ID NO: 5 and 6) was exchanged by BDNF (UniProt # P23560; SEQ ID NO: 3 and 4) signal peptide (BDNF-pro-IGF1) in the mRNA construct. This sequence consisted of the sequence encoding the pre-domain (signaling peptide) of human BDNF with 18 amino acids (nucleotides 1-54; SEQ ID NO: 4) and the sequence encoding the human IGF1 pro-domain with 27 amino acids (nucleotides 55-135; SEQ ID NO: 1). Furthermore, the construct contained the sequence encoding the full coding sequence of mature human IGF1 with 70 amino acids (nucleotides 136-348; SEQ ID NO: 2). No C-terminal E-domain was added to the construct. Figure 1 illustrates the DNA and RNA sequence of IGF1 encoded by BDNFpre-domain (signalling peptide) and IGF1 pro domain and coding domain. The respective sequence is gene synthesized in two different vector backbone pMA-T (Ampicillin resistance; SEQ ID NO: 9) and pMK (Kanamycin resistance; SEQ ID NO: 10), and were defined as Cpd.1A and Cpd.1B, respectively. The Cpd.1A construct further includes T7 promoter sequence (5' TAATACGACTCACTATA 3'; SEQ ID NO: 11) and Kozak sequence upstream of the IGF1 sequence for RNA polymerase binding and successful *in vitro* transcription (IVT) of the gene of interest. Likewise, the Cpd.1B construct includes T7 promoter sequence and Kozak sequence upstream of the IGF1 sequence for IVT of the gene of interest. In contrast to Cpd.1A, the Cpd.1B construct comprises a poly-A tail of 120 bp length into the vector to mimic eukaryotic RNA and to provide stability to mRNA. The *XhoI* and *BspQI* restriction sites were incorporated immediately after the poly-A tail for the linearization of the plasmid. In summary, the Cpd.1B synthetic mRNA construct contained an insert of the human BDNF pre-domain (signalling peptide), pro-IGF1 domain and coding (mature) IGF1 DNA without E-peptide information along with 120 bp poly-A tail. The open reading frame of the BDNF pre-domain (signalling peptide), pro-IGF1 domain and coding (mature) IGF1 DNA sequences of Cpd.1A and Cpd.1B (including poly-A tail and restriction sites) were codon optimized using GeneOptimizer algorithm and gene synthesized in respective vector backbone from GeneArt, Germany (www.thermofisher.com). The DNA sequence of the entire vectors of Cpd.1A and Cpd.1B are given in Figure 2.

### In vitro transcription (IVT) of Cpd.1A and Cpd.1B mRNA

For the generation of Cpd.1A mRNA from pMA-T vector, a transcription template was generated by PCR using mRNA forward primer 5' GCTGCAAGGCGATTAAGTTG 3' and mRNA reverse primer 5' U (2'OMe) U(2'OMe) U (2'OMe) T₍₁₁₇₎ CAGCTATGACCATGTTAATGCAG 3' (SEQ ID NO: 12 and 13). The reverse primer contained 120 bp poly-T to include a poly-A tail into the mature mRNA. In case of Cpd.1B mRNA production, the pMK vector containing Cpd.1B (also encoded poly-A tail of 120 bp length) was linearized downstream of the poly-A tail with *XhoI* enzyme. Both PCR amplicons and linearized plasmids were used as templates for IVT performed by T7 RNA polymerase in the MEGAscript T7 kit at 37°C for 2 hours (www.thermofisher.com). All mRNAs were produced co-transcriptionally with an anti-reverse CAP analog (ARCA; [m₂^{7,3'-O}G(5')ppp(5')G]) in the 5' end and chemically modified with 100% N1-methylpseudo-UTP (www.jenabioscience.com). *In vitro* transcribed mRNAs were purified using the MEGAclear kit (www.thermofisher.com) and quantified using Nanophotometer-N60 (Implen). The integrity of IVT RNA were fragment analyzed using RNA 6000 Nano kit in an Agilent 2100 Bioanalyzer (www.agilent.com).

### Pharmacokinetics of Cpd.1A after application into rat TA muscle or urethral tissue

For assessment of Cpd.1A exposure in rat tibialis anterior (TA) muscle, two injections of each 15 µl Cpd.1A at different doses (1, 3 or 10 µg per injection) into TA muscles of male or female rats were performed in adult rats. After 6, 24, 48 and 72 hours, TA muscles were harvested, divided into two parts to analyse RNA and protein content, and Cpd.1A RNA content was analysed by qPCR and IGF1 protein levels by human-specific IGF1 ELISA (Cat. # E20, Mediagnost, Reutlingen, Germany). Total RNA was extracted from flash frozen TA muscle tissues (ca. 10 mg) using Norgen's Animal Tissue RNA Purification Kit (Cat.# 25700). A TaqMan assay was developed and validated to quantify Cpd.1A and Cpd.1B RNA from muscle tissues through absolute quantification. Both forward primer (5' CGGTCTGAGGAGCCCTTCTAG 3'; SEQ ID NO: 14) and reverse primer (5' CGACAGAGGCTTCTACTTCAACAAG 3'; SEQ ID NO: 15) along with labelled probe with quencher (5' FAM-CTGCTGCCGTAGCCTG-MGB-Q500 3'; SEQ ID NO: 16) were designed to be specifically bind to Cpd.1A and Cpd.1B RNA and ordered (www.microsynth.com). To generate the standard curve, Cpd.1A mRNA dilutions ranging from 10 copies to 1 x 10⁹ copies were prepared and tested in qPCR using qScript^{™} XLT One-Step-RT-qPCR ToughMix (www.qunatabio.com) in Roche Light Cycler 480 including target samples. The observed C_{T} values plotted against the known copy number. The Cpd.1A RNA copy numbers in TA muscles were assessed by calculating unknown values from standard curves. From qPCR data, half-life was calculated using a monoexponential decay function. For assessment of Cpd.1B exposure in rat urethra, Cpd.1B (30 µg) was injected under isoflurane anesthesia at 30 µg in female rats into the urethra at two different positions approximately at the 5 and 7 o'clock positions using a Hamilton syringe (10 µl volume slowly injected). After 24 and 72 hours, tissues were harvested, and Cpd.1B content was analysed by qPCR as specified above where standard curve template generated from Cpd.1B mRNA.

### Rat vaginal distension model for stress urinary incontinence

The urethra is a conduit for elimination of urine from the bladder. It is composed of an epithelium lining the lumen, lamina propria with a prominent vascular plexus, smooth muscle and an outer layer of striated muscle (called external urethral sphincter). The urethra functions in coordination with the bladder: it remains closed during bladder filling to maintain continence and it relaxes during voiding to allow flow of urine. These processes are controlled by parasympathetic, sympathetic and somatic innervation. Parasympathetic nerves release nitric oxide to relax the urethra during bladder voiding, while sympathetic nerves release noradrenaline to contract the urethra during bladder filling. The striated muscle receives somatic innervation that contracts the muscle ensuring continence during bladder filling.

Using the model of stress urinary incontinence (SUI) in female rats the hypothesis was tested if Cpd.1B can be effective against SUI in the vaginal distension (VD) model by facilitating muscle growth and regeneration. Thereby, the effects of Cpd.1B (locally injected into the urethra once on Day 0) were compared to continuous oral treatment with the reference drug (duloxetine) on Leak Point Pressure and histological measures.

Vaginal Distension (VD) was applied for 4 hours to female Sprague-Dawley Female rats under isoflurane anesthesia. 1 hour after the end of VD, the abdomen was surgically opened to access the urethra and rats received two injections of vehicle or Cpd.1B (10 µg each) through a Hamilton syringe at approximately 5 and 7 o'clock positions. Sham treated animals (catheter without inflation) were treated with vehicle as controls. Duloxetine was administered orally once a day at 20 mg/kg to animals starting on the day of VD.

### Assessment of Leak Point Pressure (LPP) in rats after vaginal distension

For assessment of LPP rats were anesthetized with isoflurane on days 4, 9 and 14 post-VD for sedation and cannulated through the jugular vein for urethane anesthesia (1.2 g/kg, i.v.). Rats were then put in a supine position on a heated pad at 37°C. The bladder was emptied manually and filled with room temperature saline through the bladder catheter at 3.0 ml/h. After bladder filling with 0.4-0.5 ml, the abdomen was slowly depressed manually to increase abdominal pressure until fluid leakage was observed from the urethral orifice. LPP was tested 6 times on each animal (10 animals per group), at 2 minutes interval, and the mean LPP value was used for statistical analyses.

### Histological assessment of urethral integrity after VD

From 2 animals of each group, the urethra-vaginal tissues were harvested (cross-section of the mid-urethra and anterior vagina) on Day 9 and 14, and the tissues immersed in neutral buffered formalin containing 4% formaldehyde for a period of 4 h, then embedded in paraffin. Sections of 5 µm were stained with the standard procedures of Hemalun-Eosin (HE), Masson Trichrome (MT) and Picrosirius red. Based on morphology and tissue integrity, histology scores were allocated to the outer circular muscle layer (OCML), the inner longitudinal muscle layer (ILML) and the endomysial tissue of mid-urethral sections, and Day 9 and 14 sampled. The following histological scores were applied to the different layers and averaged per group:
- Outer Circular Muscle Layer (OCML, skeletal muscle):
   ∘ 0=circumferential regular
   ∘ 1=circumferential focally thinned
   ∘ 2=discontinued
   ∘ 3=mostly absent
- Inner Longitudinal Muscle Layer (ILML, smooth muscle):
   ∘ 0=mid-sized to large fascicles
   ∘ 1=small fascicles)
- Endomysial tissue:
   ∘ 0=thin, dense
   ∘ 1=thickening by loose connective tissue
   ∘ 2=focal replacement of muscles by connective tissue
   ∘ 3=replacement of muscles by connective tissue

### RESULTS

### In vitro transcription of mRNA

For different in vivo studies, Cpd.1A and Cpd.1B mRNAs were produced in 0.5-10 mg scales. The integrity of RNA confirmed by fragment analysis as a quality control measure.

### Pharmacokinetics of Cpd.1A after application into rat TA muscle

Cpd.1A exposure in rat tibialis anterior (TA) muscle was assessed 6, 24, 48 and 72 hours after two simultaneous injections of each 15 µl Cpd.1A at different doses (1, 3 or 10 µg per injection). Highest levels were observed at 6 hours with continuous decline in Cpd. 1A exposure over 72 hours (Figure 3). Thereby, an approximately linear increase was observed between the three different doses. The data suggest relevant exposure of Cpd.1A in TA muscle tissue after i.m. application over up to 72 hours. From the data obtained for the 3 and 10 µg doses, half-life was calculated using a monoexponential decay function, and half-lifes of 21 and 17 hours were estimated for the 3 and 10 µg dose, respectively (Figure 4). The data are in line with a relevant Cpd.1A exposure for up to 72 hours after i.m. application.

To analyse the relation between Cpd.1A exposure and IGF1 protein expression, data from Figure 3 were compared with the amount of IGF1 protein measured in TA muscle specimen by a human-specific IGF1 ELISA. Data for the 3 and 10 µg doses showed that after a delay of several hours after the Cpd.1A injection, a decent IGF1 protein production was observed with therapeutically relevant concentrations in muscle tissue over up to 72 hours (Figure 5). The data suggest a physiologically relevant expression of IGF1 protein over up to 72 hours after i.m. application of Cpd.1.

### Pharmacokinetics of Cpd.1B after application into rat urethral tissue

After intra-urethral injection of Cpd.1B (30 µg) under isoflurane anesthesia, a decent Cpd.1B exposure was measured 24 hours after injection, with a further decline after 72 hours (Figure 6). The data suggest a relevant exposure of Cpd.1B after intra-urethral injection over up to 72 hours.

### Rat vaginal distension model for stress urinary incontinence

For assessment of urinary incontinence in the vaginal distension (VD) animal model of stress urinary incontinence (SUI), leak point pressure (LPP) of urinary bladder was evaluated on Day 4, 9 and 14 after VD. Sham vehicle treated animals without VD were used as controls to assess baseline LPP (grey area in Figure 7). VD induced a decline of LPP within 4 days and a recovery to healthy levels after 9 and 14 days (Figure 7). In contrast, a single Cpd.1B treatment at 10 µg on Day 0 after the VD procedure led to a significant increase in LPP (*, p<0.05, student's t-test) on Day 4 that further increased up to Day 9. On Day 14, full recovery to healthy LPP levels was observed. In contrast, Duloxetine daily oral treatment at 20 mg/kg resulted in an initial increase of LPP on Day 4 (**, p<0.01, student's t-test) that continuously declined over Day 9 to control levels on Day 14 (Figure 7), suggesting a different mechanism of Duloxetine as compared to IGF1.

Evaluation of the area under curve (AUC) for all groups indicated a decrease in AUC for the VD vehicle group whereas both Cpd.1B and Duloxetine treatment groups showed a clear increase in AUC over 14 days (Figure 8).

Statistical analysis of the LPP data obtained on Day 4 and 9 (Figure 9A and 9B) showed a significant increase of LPP on Day 4 for both Cpd.1B and Duloxetine (*, p<0.05, **, p<0.01, Student's t-test) as compared to the VD vehicle treated group. Due to full recovery in the VD vehicle group, the Cpd.1B did not reach significance on Day 9.

The data suggest that both Cpd.1B and Duloxetine improved urinary bladder function although suggesting different biological mechanisms. Whereas Duloxetine exerts its benefits mainly acutely and via neuromodulatory symptomatic short-term benefit, IGF11 data suggested a continuous regenerating long-term effect on muscle tissue in the urethra.

### Histological assessment of urethral tissue after vaginal distension (VD)

Main urethral remodeling was observed within mucosa muscularis and resulted in semiquantitative changes observed in three different layers of the urethra, the outer circular skeletal muscle layer (OCML), the inner longitudinal smooth muscle layer (ILML), and the endomysial tissue increasing in size upon muscle cell damage. The assessment of histological scores revealed for the OCML that a trend was observed for an increased OCML disruption as evidenced by a slightly increased OCML score after VD resulting from more discontinued layer organisation (Figure 10). Whereas Cpd.1B treatment prevented this trend, Duloxetine treatment further deteriorated the OCML pathology (Figure 10). For the ILML consisting of smooth muscle cells, a similar trend was observed, with VD resulting in a slight increase in score, facing more small fascicles and therefore ILML disruption, and Cpd.1B preventing this disruption, whereas Duloxetine further increased the ILML score and worsened the phenotype (Figure 11). Finally, VD led to an increased replacement of skeletal as well as smooth muscle tissue by connective tissue in the endomysial tissue after 9 and 14 days, and Cpd.1B treatment completely prevented this impairment (Figure 12). In contrast, Duloxetine further worsened this pathology.

Taken together, the histological evaluations clearly indicate a different mechanism for IGF1 and Duloxetine mediated functional improvements of LPP after VD. Whereas IGF1 apparently resulted in preserving skeletal as well as smooth muscle and thereby tissue organisation and prevention of connective tissue generation, Duloxetine exerted its symptomatic effects via different mechanism.

## Claims

1. A mRNA or a therapeutic composition thereof for use in a method of treating lower urinary tract symptoms (LUTS), wherein the mRNA comprises a nucleic acid sequence encoding insulin-like growth factor 1 (IGF1).

2. The mRNA or the therapeutic composition thereof for use of claim 1, wherein the mRNA comprises a nucleic acid sequence encoding a signal peptide, optionally a nucleic acid sequence encoding the propeptide of IGF1 and a nucleic acid sequence encoding the mature IGF1.

3. The mRNA or the therapeutic composition thereof for use of claim 2, wherein the nucleic acid sequence encoding the signal peptide encodes the signal peptide of the brain-derived neurotrophic factor (BDNF).

4. The mRNA or the therapeutic composition thereof for use of claim 1, wherein the mRNA comprises a nucleic acid sequence encoding the signal peptide of the brain-derived neurotrophic factor (BDNF), a nucleic acid sequence encoding the propeptide of human IGF1, a nucleic acid sequence encoding the mature human IGF1 and does not comprise a nucleic acid sequence encoding an E-peptide of human IGF1.

5. The mRNA or the therapeutic composition thereof for use of claim 1, wherein the mRNA comprises a nucleic acid sequence as shown in SEQ ID NO: 8.

6. The mRNA or the therapeutic composition thereof for use of any one of claims 1 to 5, wherein the lower urinary tract symptoms (LUTS) are selected from the group consisting of stress urinary incontinence (SUI), mixed urinary incontinence, urge urinary incontinence, under active bladder, overflow incontinence and pelvic organ prolapse.

7. The mRNA or the therapeutic composition thereof for use of any one of claims 1 to 5, wherein the lower urinary tract symptoms (LUTS) are selected from the group consisting of stress urinary incontinence (SUI), mixed urinary incontinence and urge urinary incontinence.

8. The mRNA or the therapeutic composition thereof for use of any one of claims 1 to 5, wherein the lower urinary tract symptoms (LUTS) is stress urinary incontinence (SUI).

9. The mRNA or the therapeutic composition thereof for use of any one of claims 1 to 8, wherein the mRNA is administered to the urethral sphincter muscle of a subject.

10. The mRNA or the therapeutic composition for use of any one of claims 1 to 8, wherein the mRNA is administered to the urethral sphincter muscle of a subject by intramuscular injection.

## Patentansprüche

1. Eine mRNA oder eine therapeutische Zusammensetzung davon zur Verwendung in einem Verfahren zur Behandlung von Symptomen des unteren Harntrakts (LUTS), wobei die mRNA eine Nukleinsäuresequenz umfasst, die den insulinähnlichen Wachstumsfaktor 1 (IGF1) kodiert.

2. Die mRNA oder die therapeutische Zusammensetzung davon zur Verwendung nach Anspruch 1, wobei die mRNA eine Nukleinsäuresequenz, die ein Signalpeptid kodiert, optional eine Nukleinsäuresequenz, die das Propeptid von IGF1 kodiert, und eine Nukleinsäuresequenz, die das reife IGF1 kodiert, umfasst.

3. Die mRNA oder die therapeutische Zusammensetzung davon zur Verwendung nach Anspruch 2, wobei die Nukleinsäuresequenz, die das Signalpeptid kodiert, das Signalpeptid des vom Gehirn stammenden neurotrophen Faktors (BDNF) kodiert.

4. Die mRNA oder die therapeutische Zusammensetzung davon zur Verwendung nach Anspruch 1, wobei die mRNA eine Nukleinsäuresequenz, die das Signalpeptid des vom Gehirn stammenden neurotrophen Faktors (BDNF) kodiert, eine Nukleinsäuresequenz, die das Propeptid des humanen IGF1 kodiert, eine Nukleinsäuresequenz, die das reife humane IGF1 kodiert, umfasst und keine Nukleinsäuresequenz umfasst, die ein E-Peptid des humanen IGF1 kodiert.

5. Die mRNA oder die therapeutische Zusammensetzung davon zur Verwendung nach Anspruch 1, wobei die mRNA eine Nukleinsäuresequenz wie in SEQ ID NO: 8 gezeigt umfasst.

6. Die mRNA oder die therapeutische Zusammensetzung davon zur Verwendung nach einem der Ansprüche 1 bis 5, wobei die Symptome des unteren Harntrakts (LUTS) ausgewählt sind aus der Gruppe bestehend aus Belastungsharninkontinenz (SUI), gemischter Harninkontinenz, Drangharninkontinenz, unteraktiver Blase, Überlaufinkontinenz und Beckenorganprolaps.

7. Die mRNA oder die therapeutische Zusammensetzung davon zur Verwendung nach einem der Ansprüche 1 bis 5, wobei die Symptome des unteren Harntrakts (LUTS) ausgewählt sind aus der Gruppe bestehend aus Belastungsharninkontinenz (SUI), gemischter Harninkontinenz und Drangharninkontinenz.

8. Die mRNA oder die therapeutische Zusammensetzung davon zur Verwendung nach einem der Ansprüche 1 bis 5, wobei die Symptome des unteren Harntrakts (LUTS) Belastungsharninkontinenz (SUI) sind.

9. Die mRNA oder die therapeutische Zusammensetzung davon zur Verwendung nach einem der Ansprüche 1 bis 8, wobei die mRNA dem Harnröhrenschließmuskel eines Subjekts verabreicht wird.

10. Die mRNA oder die therapeutische Zusammensetzung zur Verwendung nach einem der Ansprüche 1 bis 8, wobei die mRNA dem Harnröhrenschließmuskel eines Subjekts durch intramuskuläre Injektion verabreicht wird.

## Revendications

1. ARNm ou composition thérapeutique de celui-ci pour utilisation dans une méthode de traitement de symptômes du bas appareil urinaire (SBAU), dans lequel l'ARNm comprend une séquence d'acide nucléique codant pour le facteur de croissance analogue à l'insuline 1 (IGF1).

2. ARNm ou composition thérapeutique de celui-ci pour utilisation selon la revendication 1, dans lequel l'ARNm comprend une séquence d'acide nucléique codant pour un peptide signal, éventuellement une séquence d'acide nucléique codant pour le propeptide de l'IGF1 et une séquence d'acide nucléique codant pour l'IGF1 mature.

3. ARNm ou composition thérapeutique de celui-ci pour utilisation selon la revendication 2, dans lequel la séquence d'acide nucléique codant pour le peptide signal code pour le peptide signal du facteur neurotrophique dérivé du cerveau (BDNF).

4. ARNm ou composition thérapeutique de celui-ci pour utilisation selon la revendication 1, dans lequel l'ARNm comprend une séquence d'acide nucléique codant pour le peptide signal du facteur neurotrophique dérivé du cerveau (BDNF), une séquence d'acide nucléique codant pour le propeptide de l'IGF1 humain, une séquence d'acide nucléique codant pour l'IGF1 humain mature et ne comprend pas de séquence d'acide nucléique codant pour un peptide E de l'IGF1 humain.

5. ARNm ou composition thérapeutique de celui-ci pour utilisation selon la revendication 1, dans lequel l'ARNm comprend une séquence d'acide nucléique telle que représentée dans SEQ ID NO : 8.

6. ARNm ou composition thérapeutique de celui-ci pour utilisation selon l'une quelconque des revendications 1 à 5, dans lequel les symptômes du bas appareil urinaire (SBAU) sont choisis dans le groupe constitué par l'incontinence urinaire d'effort (IUE), l'incontinence urinaire mixte, l'incontinence urinaire par impériosité, la vessie hypoactive, l'incontinence par regorgement et le prolapsus des organes pelviens.

7. ARNm ou composition thérapeutique de celui-ci pour utilisation selon l'une quelconque des revendications 1 à 5, dans lequel les symptômes du bas appareil urinaire (SBAU) sont choisis dans le groupe constitué par l'incontinence urinaire d'effort (IUE), l'incontinence urinaire mixte et l'incontinence urinaire par impériosité.

8. ARNm ou composition thérapeutique de celui-ci pour utilisation selon l'une quelconque des revendications 1 à 5, dans lequel les symptômes du bas appareil urinaire (SBAU) sont l'incontinence urinaire d'effort (IUE).

9. ARNm ou composition thérapeutique de celui-ci pour utilisation selon l'une quelconque des revendications 1 à 8, dans lequel l'ARNm est administré au muscle sphincter urétral d'un sujet.

10. ARNm ou composition thérapeutique pour utilisation selon l'une quelconque des revendications 1 à 8, dans lequel l'ARNm est administré au muscle sphincter urétral d'un sujet par injection intramusculaire.
